# EUROPEAN PATENT SPECIFICATION

(11) **EP 1 984 440 B1**
(45) Date of publication and mention of the grant of the patent: **03.11.2010**
(21) Application number: 07704342.0
(22) Date of filing: 05.02.2007
(51) Int. Cl.: C08K 5/42, C08K 5/00, C08L 69/00, C08K 5/3475, C08K 5/13, C08K 5/353

(54) **AROMATIC SULPHONATE FLAME RETARDANT COMPOSITIONS**
AROMATISCHE FLAMMENHEMMENDE SULFONATZUSAMMENSETZUNGEN
COMPOSITIONS IGNIFUGES A BASE DE SULFONATE AROMATIQUE

(30) Priority: 14.02.2006 EP 06101621; 17.07.2006 EP 06117299
(43) Date of publication of application: 29.10.2008
(73) Proprietor: BASF SE, 67056 Ludwigshafen (DE)
(72) Inventor: BREINER, Christine, 69514 Laudenbach (DE); EHLIS, Thomas, 79100 Freiburg (DE); HOCHBERG, Robert, 79249 Merzhausen (DE); KNIESEL, Simon, 69221 Heidelberg-Dossenheim (DE)
(86) International application number: PCT/EP2007/051058
(87) International publication number: WO 2007/093513

(56) References cited:
- WO-A-2005/030857
- WO-A-2006/087241
- DE-A1- 1 794 386
- US-A- 4 263 201
- US-A1- 2005 143 501
- US-B1- 6 174 854
- US-B1- 6 238 804

## Description

The invention relates to flame retardant compositions comprising salts of selected aromatic sulphonic acids and polycarbonates and to a process for imparting flame retardancy to a polymer substrate comprising polycarbonates and salts of selected aromatic sulphonic acids.

Polycarbonates are thermoplastic polymers of high toughness, outstanding transparency, excellent compatibility with several polymers, and high heat distortion resistance. Polycarbonates correspond to the general formula

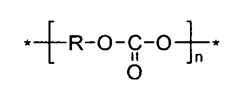

The economically most important polycarbonate is 2,2-bis(4-hydroxyphenyl)propane polycarbonate (1), also termed bisphenol A polycarbonate [24936-68-3] (BPA-PC): 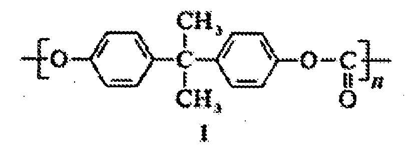 cf. Ullmann's Encyclopaedia of Industrial Chemistry, On-Line Edition, Wiley-VCH*, DOI: 10.1002*/*14356007.a21_207,* and entry *Polycarbonate, Roempp On-line, www.roempp.com.*

Various additives for improving the mechanical, chemical and thermal properties of polycarbonates are known. Fluorocarbon terminated poly-carbonates are useful for various technical applications, such as reducers of surface energy, "surface modifiers", for organic materials, preferably polycarbonates, polyesters, polyacrylates or polymethacrylates or their mixtures, blends or alloys. Polymers with such a reduced surface energy possess desirable properties, such as "easy to clean", "self-cleaning", "antisoiling", "soil-release", "antigraffiti", "oil resistance", "solvent resistance", "chemical resistance", "self lubricating", "scratch resistance", "low moisture absorption" and "hydrophobic" surface. The preparation of particularly useful fluorocarbon terminated polycarbonates is described in the International Patent Application No. PCT/EP2004/053331*.*

Flame retardants are added to polymeric materials (synthetic or natural) to enhance the flame retardant properties of the polymers. Depending on their composition, flame retardants may act in the solid, liquid or gas phase either chemically, e.g. as a spumescent by liberation of nitrogen, and/or physically, e.g. by producing a foam coverage. Flame retardants interfere during a particular stage of the combustion process, e.g. during heating, decomposition, ignition or flame spread.

The addition of flame retardants to polycarbonates is known, cf. J. Troitzsch, Plastics Flammability Handbook, 3rd edition, Hanser Publishers, Munich 2004, pp. 158-172 (ISBN 3-446-21308-2*).*

Alkali metal, earth alkali metal or ammonium salt-based flame retardants are particularly suitable at low concentrations. Among these salts, perfluoroalkane sulphonates belong to the more efficient ones. Their use as flame retardants in polycarbonates has been known; cf. T. Ishikawa et al., Journal of Macromolecular Science, Part A-Pure and Applied Chemistry, Vol. A41, No.5, pp.523-535, 2004*.*

In applications where a sample thickness smaller or equal than 1.6 mm is required, a flame retardancy of V-0, according to UL-94 (Underwriter's Laboratories Subject 94), is obtained by the addition of a so-called anti-dripping agent, such as polytetrafluoroethylene: Other co-additives for flame retardants have been proposed, such as haloarylphosphates, cf. U.S. Patent Specification No. 5,478,874 or guanidine salts; cf. U.S. Patent Specification No. 6,518,340*.* The addition of polysiloxanes of different structures has been proposed in various references; cf. U. S. Patent Specification Nos. 6,660, 787,6*,* 727,302 or 6,730,720*.* A problem of these additives is seen in the fact that the concentration of the flame retardant must be increased to arrive at the V-0 classification, which is detrimental to the mechanical, chemical and thermal properties of polycarbonates.

Therefore, the present invention relates to finding suitable additives, which are applicable in low concentrations as flame retardants in polycarbonates. It has surprisingly been found that salts of selected aromatic sulphonic acids are particularly suitable additives even at low concentrations. Like the flame retardants of first choice, such as alkali metal, earth alkali metal or ammonium salt-based flame retardants, the salts of selected aromatic sulphonic acids are present in small quantities in the polycarbonates and, due to the low dosing levels, have no significant negative effect on polymer mechanics and other properties.

The present invention relates to a composition, particularly a flame retardant composition, which comprises
a) At least one salt of an aromatic sulphonic acid selected from the group consisting of
   a₁) Triazinylaminostilbene disulphonic acid salts of the formula 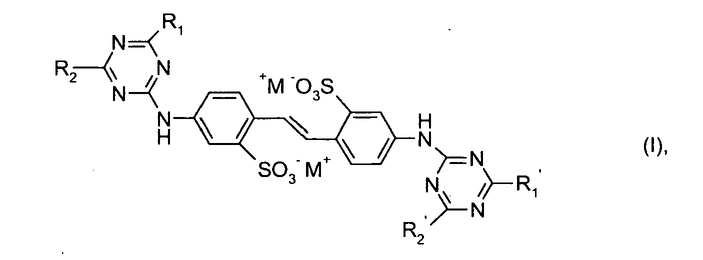 Wherein
      R₁ and R₂ independently of one another represent -NH₂, -NHC₁-C₄alkyl, -N(C₁-C₄alkyl)₂, -NH-hydroxyC₂-C₄alkyl, -N(hydroxy-C₂-C₄alkyl)₂, -N(C₁-C₄alkyl)(hydroxy-C₂-C₄alkyl), phenylamino, diphenylamino, phenylamino or diphenylamino substituted by 1-3 substituents selected from the group consisting of -S(=O)₂-O⁻ M⁺, C₁-C₄alkyl and C₁-C₄alkoxy, or 5- or 6-membered saturated N-heterocyclyl;
      R₁' and R₂ are as defined as R₁ and R₂; and
      M⁺ represents an alkali metal atom, ammonium or a cation formed from an amine;
   a₂) 2-(2-Hydroxyphenyl)-2H-benzotriazolylsulphonic acid salts of the formula 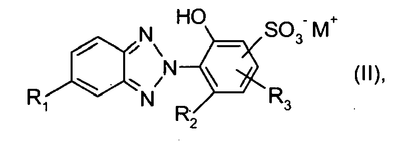 Wherein
      R₁ represents hydrogen, C₁-C₄alkyl or the group -S(=O)₂-O⁻ M⁺;
      R₂ represents hydrogen or hydroxy;
      R₃ represents hydrogen, C₁-C₄alkyl, C₁-C₄alkoxy or benzyl; and
      M⁺ represents an alkali metal atom, ammonium or a cation formed from an amine;
   a₃) Sulphophenyl substituted bis-stilbene derivatives of the formula 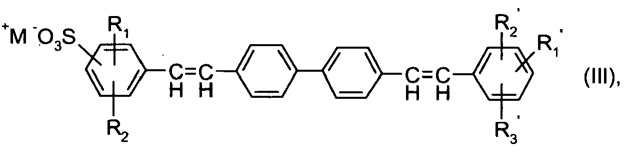 Wherein
      R₁ and R₂ independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy;
      R₁', R₂' and R₃ independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy; and
      M⁺ represents an alkali metal atom, ammonium or a cation formed from an amine; and
   a₄) Sulpho substituted 2,5-bis-(benzoxazolyl)-thiophene derivatives of the formula: 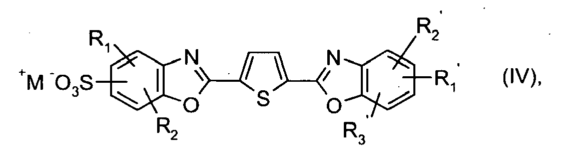 Wherein
      R₁ and R₂ independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy;
      R₁', R₂ and R₃' independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy; and
      M⁺ represents an alkali metal atom, ammonium or a cation formed from an amine;
   a₅) Sulpho substituted phenol derivatives of the formula 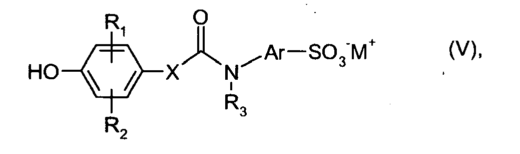 Wherein
      R₁ and R₂ independently of one another represent hydrogen or a substituent selected from the group consisting of C₁-C₄alkyl, C₅-C₆cycloalkyl, C₅-C₆cycloalkyl-C₁-C₄alkyl, aryl and aryl-C₁-C₄alkyl;
      R₃ represents hydrogen or C₁-C₄alkyl;
      X represents C₂-C₆alkylene;
      Ar represents phenyl, phenyl substituted by 1 or 2 substituents selected from the group consisting of C₁-C₄alkyl, C₅-C₆cycloalkyl, C₅-C₆cydoalkyl-C₁-C₄alkyl, aryl and aryl-C₁-C₄alkyl, naphthyl or naphthyl substituted by 1 - 4 C₁-C₄alkyl; and M⁺ represents an alkali metal atom, ammonium or a cation formed from an amine;
      and as an additional component anti-dripping agents; and b) a polymer substrate comprising polycarbonate or polycarbonate blends.

A preferred embodiment of the invention relates to a composition, particularly a flame retardant composition, which comprises
a) At least one salt of an aromatic sulphonic acid selected from the group consisting of
   a₁) Triazinylaminostilbene disulphonic acid salts (I), wherein
      R₁ and R₂ independently of one another represent -NH₂, -N(C₁-C₄alkyl)₂, phenylamino substituted by 1-3 substituents selected from the group consisting of -S(=O)₂-O⁻ M⁺, C₁-C₄alkyl and C₁-C₄alkoxy, or 5- or 6-membered saturated N-heterocyclyl;
      R₁' and R₂ are as defined as R₁ and R₂; and
      M⁺ represents an alkali metal atom;
   a₂) 2-(2-Hydroxyphenyl)-2H-benzotriazolylsulphonic acid salts (II), wherein
      R₁ represents hydrogen;
      R₂ represents hydrogen;
      R₃ represents hydrogen or C₁-C₄alkyl; and
      M⁺ represents an alkali metal atom;
   a₃) Sulphophenyl substituted bis-stilbene derivatives (III), wherein
      R₁ and R₂ independently of one another represent hydrogen or C₁-C₄alkyl; One of R₁', R₂' and R₃ represents -S(=O)₂-O⁻ M⁺ and the other ones represent hydrogen or C₁-C₄alkyl; and
      M⁺ represents an alkali metal atom;
   a₄) Sulpho substituted 2,5-bis-(benzoxazolyl)-thiophene derivatives (IV), wherein
      R₁ and R₂ independently of one another represent hydrogen or C₁-C₄alkyl;
      R₁', R₂' and R₃ independently of one another represent hydrogen or a substituent selected from the group consisting of -S(=O)₂-O⁻ M⁺ and C₁-C₄alkyl; and
      M⁺ represents an alkali metal atom; and
   a₅) Sulpho substituted phenol derivatives (V), wherein
      R₁ and R₂ independently of one another represent hydrogen or C₁-C₄alkyl;
      R₃ represents hydrogen or C₁-C₄alkyl;
      X represents C₂-C₆alkylene;
      Ar represents phenyl or phenyl substituted by 1 or 2 C₁-C₄alkyl; and
      M⁺ represents an alkali metal atom; and as an additional component anti-dripping agents; and
b) A polymer substrate comprising polycarbonate or polycarbonate blends.

A particularly preferred embodiment relates to a composition, particularly a flame retardant composition, which comprises
a) At least one salt of an aromatic sulphonic acid selected from the group consisting of
   a₁) Triazinylaminostilbene disulphonic acid salts (I), wherein
      R₁ and R₂ independently of one another represent -N(C₁-C₄alkyl)₂, phenylamino substituted by 1 or 2 -S(=O)₂-O⁻ M⁺, or morpholinyl;
      R₁' and R₂' are as defined as R₁ and R₂; and
      M⁺ represents an alkali metal atom;
   a₂) 2-(2-Hydroxyphenyl)-2H-benzotriazolylsulphonic acid salts (II), wherein
      R₁ represents hydrogen or C₁-C₄alkyl;
      R₂ represents hydrogen or hydroxy;
      R₃ represents hydrogen or C₁-C₄alkyl; and
      M⁺ represents an alkali metal atom; and
   a₃) Sulphophenyl substituted bis-stilbene derivatives (III), wherein
      R₁ and R₂ independently of one another represent hydrogen or C₁-C₄alkyl;
      One of R₁', R₂' and R₃' represents -S(=O)₂-O⁻ M⁺ and the other ones represent hydrogen or C₁-C₄alkyl; and
      M⁺ represents an alkali metal atom; and
   a₅) Sulpho substituted phenol derivatives (V), wherein
      One of R₁ and R₂ represents methyl and the other one represents tert-butyl or both of R₁ and R₂ represent tert-butyl;
      R₃ represents hydrogen or methyl;
      X represents C₂-C₄alkylene;.
      Ar represents phenyl substituted by 1 methyl and 1 tert-butyl or by 2 tert-butyl; and
      M⁺ represents an alkali metal atom; and as an additional component anti-dripping agents; and
b) A polymer substrate comprising polycarbonate or polycarbonate blends.

A highly preferred embodiment relates to a composition, particularly a flame retardant composition, which comprises
a) At least one salt of an aromatic sulphonic acid selected from the group consisting of
   a₁) Triazinylaminostilbene disulphonic acid salts (I), wherein
      One of R₁ and R₂ represents -N(C₁-C₄alkyl)₂ and the other one represents phenylamino substituted by 2 -S(=O)₂-O⁻ M⁺;
      R₁' and R₂' are as defined as R₁ and R₂; and
      M⁺ represents an alkali metal atom;
   a₂) 2-(2-Hydroxyphenyl)-2H-benzotriazolylsulphonic acid salts (II), wherein
      R₁ and R₂ represent hydrogen; and
      R₃ represents hydrogen or C₁-C₄alkyl; and
      M⁺ represents an alkali metal atom; and
   a₃) Sulphophenyl substituted bis-stilbene derivatives (III), wherein
      R₁ and R₂ represent hydrogen;
      One of R₁', R₂' and R₃ represents -S(=O)₂-O⁻ M⁺ and the other ones represent hydrogen; and
   M⁺ represents an alkali metal atom;
   a₅) Sulpho substituted phenol derivatives (V), wherein
      R₁ and R₂ represent tert-butyl;
      R₃ represents hydrogen;
      X represents ethylene;
      Ar represents phenyl substituted by 2 tert-butyl; and
      M⁺ represents an alkali metal atom; and as an additional component anti-dripping agents; and
b) A polymer substrate comprising polycarbonate or polycarbonate blends.

A most preferred embodiment relates to a composition, particularly a flame retardant composition, which comprises
a) At least one salt of an aromatic sulphonic acid selected from the group consisting of
   a₁) A triazinylaminostilbene disulphonic acid salt of the formula 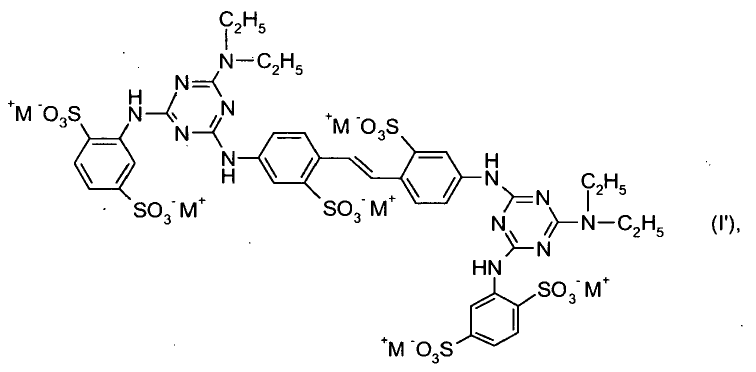
   a₂) A 2-(2-hydroxyphenyl)-2H-benzotriazolylsulphonic acid salt of the formula 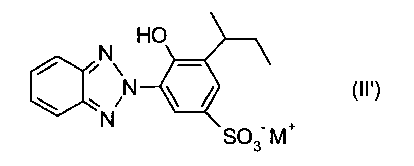
   a₃) A sulphophenyl substituted bis-stilbene derivative of the formula 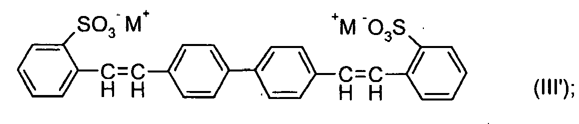 and
   a₅) A sulpho substituted phenol derivative of the formula 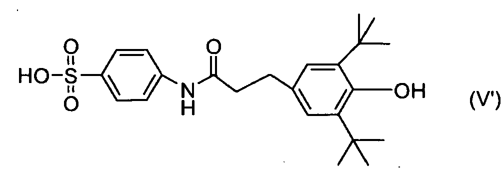 and a salt thereof;
      and as an additional component anti-dripping agents; and
b) A polymer substrate comprising polycarbonate or polycarbonate blends.

The compositions according to the invention attain the desirable V-0 rating, according to UL-94 (Underwriter's Laboratories Subject 94) and other excellent ratings in related test methods while preserving the excellent mechanical, chemical and thermal properties of polycarbonates, such as light transparency.

The composition, as defined above, comprises the following components:

### Component a)

The term salt of an aromatic sulphonic acid comprises within its scope preferably metal salts, for example an alkali metal or alkaline earth metal salt, e.g. the sodium, potassium, calcium salt.

According to an alternative embodiment, the term salts comprises non-metallic salts, e.g. the ammonium, (C₁-C₂₂alkyl)₁₋₄ammonium or (2-hydroxyethyl)₁₋₄ammonium, e.g. tetramethylammonium, tetraethylammonium or the 2-hydroxyethyltrimethylammonium salt.

Therefore the definition of the cation M+ in the formula above comprises within its scope an alkali metal atom, e.g. the sodium or potassium ion, the ammonium ion or a cation formed from an amine, e.g. (C₁-C₂₂alkyl)₁₋₄ammonium or (2-hydroxyethyl)₁₋₄ammonium, e.g. the tetramethylammonium, tetraethylammonium or the 2-hydroxyethyltrimethylammonium ions.

In a triazinylaminostilbene disulphonic acid salt (I) C₁-C₄alkyl present in R₁ and R₂ and, correspondingly in R₁' and R₂', is methyl, ethyl, n-propyl, isopropyl, n-butyl, isobutyl or tert-butyl.

Hydroxy-C₂-C₄alkyl is preferably 2-hydroxyethyl.

Phenylamino or diphenylamino substituted by 1-3 substituents selected from the group consisting of -S(=O)₂-O⁻ M⁺, C₁-C₄alkyl and C₁-C₄alkoxy is, for example, phenylamino or diphenylamino, wherein phenyl is substituted by one or two -S(=O)₂-O⁻ Na⁺ groups or by one or two methyl or methoxy groups.

C₁-C₄Alkoxy is methoxy, ethoxy, n-propoxy, isopropoxy, n-butoxy, isobutoxy or tert-butoxy. 5- or 6-membered saturated N-heterocyclyl is, for example, pyrrolidinyl, piperidinyl or, preferably, morpholinyl.

Particularly preferred is Tinopal® SFP (Ciba Specialty Chemicals): Sodium-4,4'-bis(2-amino-4-monoethanolamino-6-s-triazinyl)-diaminostilbene-2,2'-disulphonate:

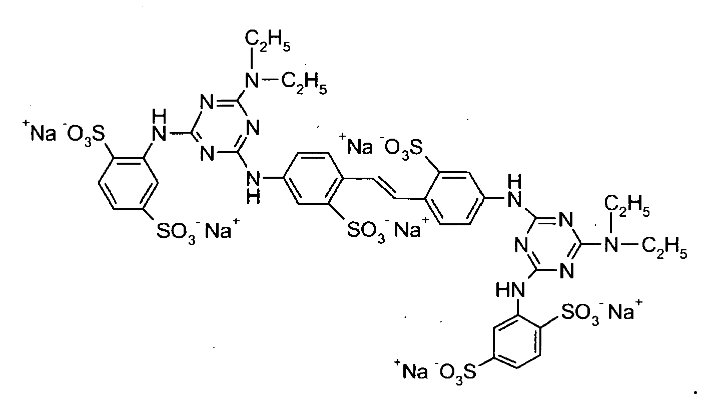

Triazinylaminostilbene disulphonic acid salts (I) are known compounds. Their technical application as fluorescent whitening agents and their preparation is described in WO 2005/068597*.* In a 2-(2-hydroxyphenyl)-2H-benzotriazolylsulphonic acid salt (II) the definitions of M⁺ C₁-C₄alkyl and C₁-C₄alkoxy present in R₁, R₂ and R₃ correspond to the definitions in compounds (I).

Particularly preferred is Tinogard® HS (Ciba Specialty Chemicals): Sodium 3-(2H-benzotriazol-2-yl)-4-hydroxy-5-(1-methylpropyl)-benzenesulphonate: 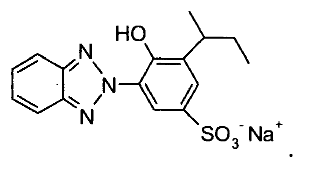 2-(2-Hydroxyphenyl)-2H-benzotriazolylsulphonic acid salts (II) are known compounds. Their technical application as photo-stabilising agents against photo-tendering in textiles and the preparation of these compounds is described in WO 84/02365*.*

In a sulphophenyl substituted bis-stilbene derivative (III) the definitions of M⁺, C₁-C₄alkyl and C₁-C₄alkoxy present in R₁ and R₂ and in R₁', R₂' and R₃' correspond to the definitions in compounds (I).

Phenyl-C₁-C₄alkyl is, for example, benzyl or 1- or 2-phenethyl.

Particularly preferred is TINOPAL CBS: Disodium 2,2'-(4,4'-biphenyldiyldi-2,1-ethene-diyl)-dibenzenesulphonate:

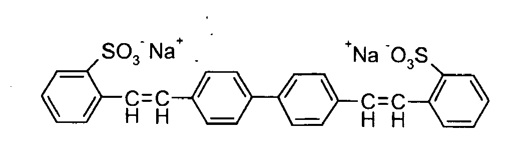

Sulphophenyl substituted bis-stilbene derivatives are known compounds. Their technical application as optical brighteners and their preparation is described in U.S. Patent Specification No. 3,984,399*.*

In a sulpho substituted phenol derivative (V) R₁ and R₂ independently of one another represent hydrogen or a substituent selected from the group consisting of C₁-C₄alkyl, C₅-C₆cycloalkyl, C₅-C₆cydoalkyl-C₁-C₄alkyl, aryl and aryl-C₁-C₄alkyl.

C₁-C₄alkyl is methyl, ethyl, n- or isopropyl or n-, iso- or tert-butyl.

C₅-C₆cycloalkyl is cyclopentyl or cyclohexyl.

C₅-C₆cydoalkyl-C₁-C₄alkyl is e.g. cyclopentylmethyl or cyclohexylmethyl.

Aryl is preferably phenyl.

Aryl-C₁-C₄alkyl is preferably benzyl or 2-phenylethyl.

R₃ is preferably hydrogen or methyl.

X defined as C₂-C₆alkylene is preferably straight chain, e.g. ethylene, n-propylene or n-butylene.

Ar defined as phenyl substituted by 1 or 2 substituents selected from the group consisting of C₁-C₄alkyl, C₅-C₆cycloalkyl, C₅-C₆cycloalkyl-C₁-C₄alkyl, is e.g. phenyl substituted by 1 methyl and 1 tert-butyl or phenyl substituted by 2 tert-butyl, phenyl substituted by cyclopentyl or cyclohexyl, phenyl substituted by benzyl, or phenyl substituted by cyclopentylmethyl or cyclohexylmethyl.

Naphthyl substituted by 1 - 4 C₁-C₄alkyl is e.g. naphthyl substituted by 1-4 methyl, naphthyl substituted by 1 methyl or 1-tert-butyl or naphthyl substituted by 2 tert-butyl.

Sulpho substituted phenol derivatives (V) are known compounds and can be prepared by known methods, such as the ones described in U.S. Patent Specification No. 3,665,031*.*

According to a preferred embodiment a sulpho substituted phenol derivative (V) is represented by the following structural formula

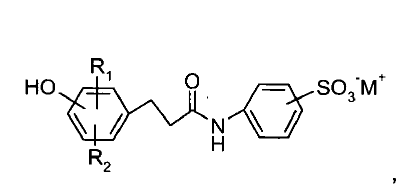

Wherein One of R₁ and R₂ represents methyl and the other one represents tert-butyl or both of R₁ and R₂ represent tert-butyl and M⁺ represents an alkali metal ion.

According to a particularly preferred embodiment a sulpho substituted phenol derivative (V) is represented by the following structural formula 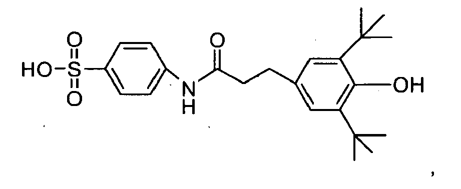 which is commercially available from Ciba Specialty Chemicals Holding Inc. (Tinogard® AO 6).

### Component b)

The polymer substrate comprising polycarbonates or polycarbonate blends may be of any grade and prepared by any known method. The term polymer substrate comprises within its scope any polycarbonate homopolymers or copolymers thereof, such as copolymers with polyesters.

Polycarbonates are thermoplastic polymers that correspond to the general formula:

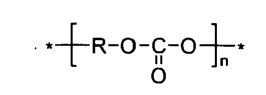

Polycarbonates are obtainable by interfacial processes or by melt processes (catalytic transesterification). The polycarbonate may be either branched or linear in structure and may include any functional substituents. Polycarbonate copolymers and polycarbonate blends are also within the scope of the invention. The term polycarbonate should be interpreted as inclusive of copolymers and blends with other thermoplastics. Methods for the manufacture of polycarbonates are known, for example, from U.S. Patent Specification Nos. 3,030,331*;* 3,169,121*;* 4,130,458*;* 4, 263,201*;* 4,286,083*;* 4,552,704*;* 5,210,268*; and* 5,606,007*.* A combination of two or more polycarbonates of different molecular weights may be used.

Preferred are polycarbonates obtainable by reaction of a diphenol, such as bisphenol A, with a carbonate source. Examples of suitable diphenols are: 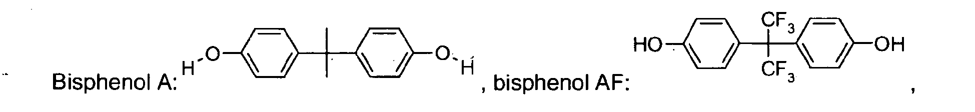 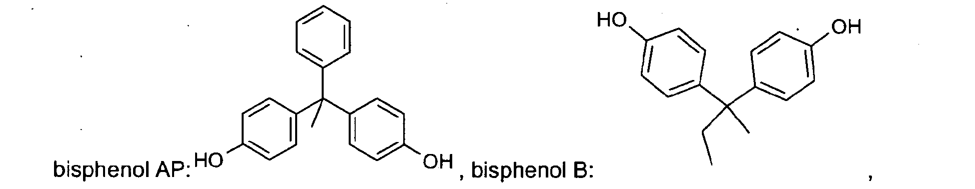 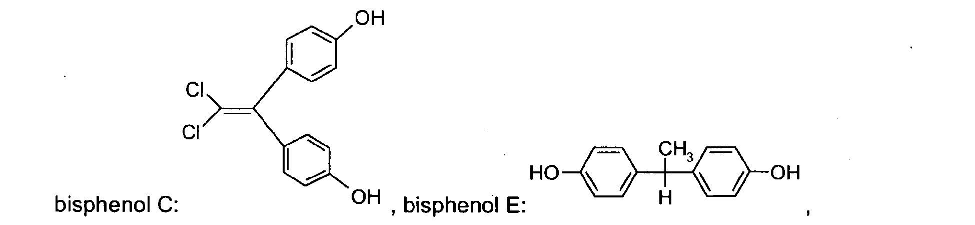 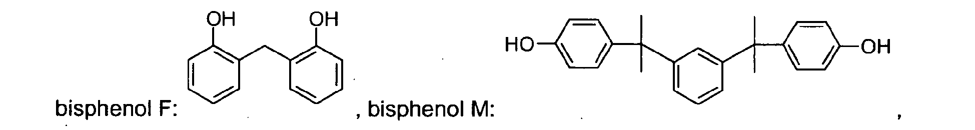 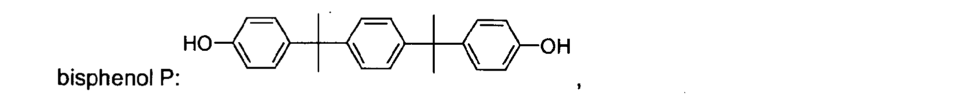 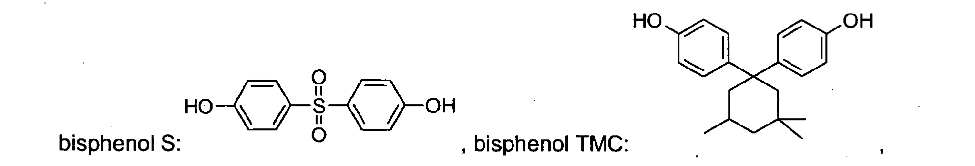 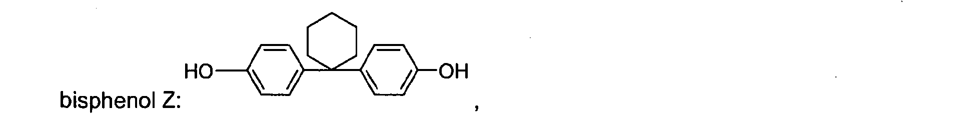 4,4'-(2-norbornylidene)bis(2,6-dichlorophenol); or
fluorene-9-bisphenol:

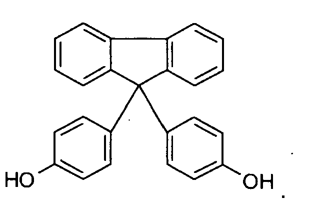

The carbonate source may be a carbonyl halide, a carbonate ester or a haloformate. Suitable carbonate halides are phosgene or carbonylbromide. Suitable carbonate esters are dialkylcarbonates, such as dimethyl- or diethylcarbonate, diphenyl carbonate, phenyl-alkylphenyl-carbonate, such as phenyl-tolylcarbonate, dialkylcarbonates, such as dimethyl- or diethylcarbonate, di-(halophenyl)carbonates, such as di-(chlorophenyl)carbonate, di-(bromophenyl)carbonate, di-(trichlorophenyl)carbonate or di-(trichlorophenyl)carbonate, di-(alkylphenyl)carbonates, such as di-tolylcarbonate, naphthylcarbonate, dichloronaphthylcarbonate and others.

Other process details, such as the addition of molecular weight regulators, acid acceptors, catalysts are disclosed in the references mentioned above.

According to an additional embodiment, the polymer substrate comprising polycarbonates or polycarbonate blends is a polycarbonate-copolymer, wherein isophthalate/terephthalate-resorcinol segments are present. Such polycarbonates are commercially available, e.g. Lexan® SLX (General Electrics Co. USA). Other polymeric substrates of component b) may additionally contain in the form as admixtures or as copolymers a wide variety of synthetic polymers including polyolefins, polystyrenes, polyesters, polyethers, polyamides, poly-(meth)acrylates, thermoplastic polyurethanes, polysulphones, polyacetals and PVC, including suitable compatibilizing agents. For example, the polymer substrate may additionally contain thermoplastic polymers selected from the group of resins consisting of polyolefins, thermoplastic polyurethanes, styrene polymers and copolymers thereof. Specific embodiments include polypropylene (PP), polyethylene (PE), polyamide (PA), polybutylene terephthalate (PBT), polyethylene terephthalate (PET), glycol-modified polycyclohexylenemethylene terephthalate (PCTG), polysulphone (PSU), polymethylmethacrylate (PMMA), thermoplastic polyurethane (TPU), acrylonitrile-butadiene-styrene (ABS), acrylonitrile-styrene-acrylic ester (ASA), acrylonitrile-ethylene-propylene-styrene (AES), styrene-maleic anhydride (SMA) or high impact polystyrene (HIPS).

A list of suitable synthetic polymers is given below:
1. Polymers of monoolefins and diolefins, for example polypropylene, polyisobutylene, poly-but-1-ene, poly-4-methylpent-1-ene, polyvinylcyclohexane, polyisoprene or polybutadiene, as well as polymers of cycloolefins, for instance of cyclopentene or norbornene, polyethylene (which optionally can be crosslinked), for example high density polyethylene (HDPE), high density and high molecular weight polyethylene (HDPE-HMW), high density and ultrahigh molecular weight polyethylene (HDPE-UHMW), medium density polyethylene (MDPE), low density polyethylene (LDPE), linear low density polyethylene (LLDPE), (VLDPE) and (ULDPE).
   Polyolefins, i.e. the polymers of monoolefins exemplified in the preceding paragraph, preferably polyethylene and polypropylene, can be prepared by different and especially by the following methods:
   a) Radical polymerisation (normally under high pressure and at elevated temperature).
   b) Catalytic polymerisation using a catalyst that normally contains one or more than one metal of groups IVb, Vb, VIb or VIII of the Periodic Table. These metals usually have one or more than one ligand, typically oxides, halides, alcoholates, esters, ethers, amines, alkyls, alkenyls and/or aryls that may be either π- or σ-coordinated. These metal complexes may be in the free form or fixed on substrates, typically on activated magnesium chloride, titanium(III) chloride, alumina or silicon oxide. These catalysts may be soluble or insoluble in the polymerisation medium. The catalysts can be used by themselves in the polymerisation or further activators may be used, typically metal alkyls, metal hydrides, metal alkyl halides, metal alkyl oxides or metal alkyloxanes, said metals being elements of groups Ia, IIa and/or IIIa of the Periodic Table. The activators may be modified conveniently with further ester, ether, and amine or silyl ether groups. These catalyst systems are usually termed Phillips, Standard Oil Indiana, Ziegler-Natta), TNZ (DuPont), metallocene or single site catalysts (SSC).
2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefins and diolefins with each other or with other vinyl monomers, for example ethylene/propylene copolymers, linear low density polyethylene (LLDPE) and mixtures thereof with low density polyethylene (LDPE), propylene/but-1-ene copolymers, propylene/isobutylene copolymers, ethylene/but-1-ene copolymers, ethylene/hexene copolymers, ethylene/methylpentene copolymers, ethylene/heptene copolymers, ethylene/octene copolymers, ethylene/vinylcyclohexane copolymers, ethylene/cycloolefin copolymers (e.g. ethylene/norbornene like COC), ethylene/1-olefins copolymers, where the 1-olefin is generated in-situ; propylene/butadiene copolymers, isobutylene/isoprene copolymers, ethylene/vinylcyclohexene copolymers, ethylene/alkyl acrylate copolymers, ethylene/alkyl methacrylate copolymers, ethylene/vinyl acetate copolymers or ethylene/acrylic acid copolymers and their salts (ionomers) as well as terpolymers of ethylene with propylene and a diene such as hexadiene, dicyclopentadiene or ethylidene-norbornene; and mixtures of such copolymers with one another and with polymers mentioned in 1) above, for example polypropylene/ethylene-propylene copolymers, LDPE/ethylene-vinyl acetate copolymers (EVA), LDPE/ethylene-acrylic acid copolymers (EAA), LLDPE/EVA, LLDPE/EAA and alternating or random polyalkylene/carbon monoxide copolymers and mixtures thereof with other polymers, for example polyamides.
4. Hydrocarbon resins (for example C₅-C₉) including hydrogenated modifications thereof (e.g. tackifiers) and mixtures of polyalkylenes and starch;
   The homopolymers and copolymers mentioned above may have a stereo structure including syndiotactic, isotactic, hemi-isotactic or atactic; where atactic polymers are preferred. Stereo block polymers are also included.
5. Polystyrene, poly (p-methylstyrene), poly(α-methylstyrene).
6. Aromatic homopolymers and copolymers derived from vinyl aromatic monomers including styrene, α-methylstyrene, all isomers of vinyl toluene, especially p-vinyl toluene, all isomers of ethyl styrene, propyl styrene, vinyl biphenyl, vinyl naphthalene, and vinyl anthracene, and mixtures thereof. Homopolymers and copolymers may have a stereo structure including syndiotactic, isotactic, hemi-isotactic or atactic; where atactic polymers are preferred. Stereo block polymers are also included;
   a) Copolymers including aforementioned vinyl aromatic monomers and comonomers selected from ethylene, propylene, dienes, nitriles, acids, maleic anhydrides, maleimides, vinyl acetate and vinyl chloride or acrylic derivatives and mixtures thereof, for example styrene/butadiene, styrene/acrylonitrile, styrene/ethylene (interpolymers), styrene/alkyl methacrylate, styrene/butadiene/alkyl acrylate, styrene/butadiene/alkyl methacrylate, styrene/maleic anhydride, styrene/acrylonitrile/methyl acrylate; mixtures of high impact strength of styrene copolymers and another polymer, for example a polyacrylate, a diene polymer or an ethylene/propylene/diene terpolymer; and block copolymers of styrene such as styrene/butadiene/styrene, styrene/isoprene/styrene, styrene/ethylene/butylene/styrene or styrene/ethylene/propylene/styrene.
   b) Hydrogenated aromatic polymers derived from hydrogenation of polymers mentioned under 6.), especially including polycyclohexylethylene (PCHE) prepared by hydrogenating atactic polystyrene, often referred to as polyvinylcyclohexane (PVCH).
   c) Hydrogenated aromatic polymers derived from hydrogenation of polymers mentioned under 6a). Homopolymers and copolymers may have a stereo structure including syndiotactic, isotactic, hemi-isotactic or atactic; where atactic polymers are preferred. Stereo block polymers are also included.
7. Graft copolymers of vinyl aromatic monomers such as styrene or α-methylstyrene, for example styrene on polybutadiene, styrene on polybutadiene-styrene or polybutadiene-acrylonitrile copolymers; styrene and acrylonitrile (or methacrylonitrile) on polybutadiene; styrene, acrylonitrile and methyl methacrylate on polybutadiene; styrene and maleic anhydride on polybutadiene; styrene, acrylonitrile and maleic anhydride or maleimide on polybutadiene; styrene and maleimide on polybutadiene; styrene and alkyl acrylates or methacrylates on polybutadiene; styrene and acrylonitrile on ethylene/propylene/diene terpolymers; styrene and acrylonitrile on polyalkyl acrylates or polyalkyl methacrylates, styrene and acrylonitrile on acrylate/butadiene copolymers, as well as mixtures thereof with the copolymers listed under 6), for example the copolymer mixtures known as ABS, MBS, ASA or AES polymers.
8. Halogen-containing polymers such as polychloroprene, chlorinated rubbers, chlorinated and brominated copolymer of isobutylene-isoprene (halobutyl rubber), chlorinated or sulphochlorinated polyethylene, copolymers of ethylene and chlorinated ethylene, epichlorohydrin homo- and copolymers, especially polymers of halogen-containing vinyl compounds, for example polyvinyl chloride, polyvinylidene chloride, polyvinyl fluoride, polyvinylidene fluoride, as well as copolymers thereof such as vinyl chloride/vinylidene chloride, vinyl chloride/vinyl acetate or vinylidene chloride/vinyl acetate copolymers.
9. Polymers derived from α,β-unsaturated acids and derivatives thereof such as polyacrylates and polymethacrylates; polymethyl methacrylates, polyacrylamides and polyacrylonitriles, impact-modified with butyl acrylate.
10. Copolymers of the monomers mentioned under 9) with each other or with other unsaturated monomers, for example acrylonitrile/ butadiene copolymers, acrylonitrile/alkyl acrylate copolymers, acrylonitrile/alkoxyalkyl acrylate or acrylonitrile/vinyl halide copolymers or acrylonitrile/ alkyl methacrylate/butadiene terpolymers.
11. Polymers derived from unsaturated alcohols and amines or the acyl derivatives or acetals thereof, for example polyvinyl alcohol, polyvinyl acetate, polyvinyl stearate, polyvinyl benzoate, polyvinyl maleate, polyvinyl butyral, polyallyl phthalate or polyallyl melamine; as well as their copolymers with olefins mentioned in 1. above.
12. Homopolymers and copolymers of cyclic ethers such as polyalkylene glycols, polyethylene oxide, polypropylene oxide or copolymers thereof with bisglycidyl ethers.
13. Polyacetals such as polyoxymethylene and those polyoxymethylenes, which contain ethylene oxide as a comonomer; polyacetals modified with thermoplastic polyurethanes, acrylates or MBS.
14. Polyphenylene oxides and sulphides, and mixtures of polyphenylene oxides with styrene polymers or polyamides.
15. Polyurethanes derived from hydroxyl-terminated polyethers, polyesters or polybutadienes on the one hand and aliphatic or aromatic polyisocyanates on the other, as well as precursors thereof.
16. Polyamides and copolyamides derived from diamines and dicarboxylic acids and/or from aminocarboxylic acids or the corresponding lactams, for example polyamide 4, polyamide 6, polyamide 6/6, 6/10, 6/9, 6/12, 4/6, 12/12, polyamide 11, polyamide 12, aromatic polyamides starting from m-xylene diamine and adipic acid; polyamides prepared from hexamethylenediamine and isophthalic or/and terephthalic acid and with or without an elastomer as modifier, for example poly-2,4,4,-trimethylhexamethylene terephthalamide or poly-m-phenylene isophthalamide; and also block copolymers of the aforementioned polyamides with polyolefins, olefin copolymers, ionomers or chemically bonded or grafted elastomers; or with polyethers, e.g. with polyethylene glycol, polypropylene glycol or polytetramethylene glycol; as well as polyamides or copolyamides modified with EPDM or ABS; and polyamides condensed during processing (RIM polyamide systems).
17. Polyureas, polyimides, polyamide imides, polyether imides, polyester imides, polyhydantoins and polybenzimidazoles.
18. Polyesters derived from dicarboxylic acids and diols and/or from hydroxycarboxylic acids or the corresponding lactones, for example polyethylene terephthalate, polybutylene terephthalate, poly-1,4-dimethylolcyclohexane terephthalate, polyalkylene naphthalate (PAN) and polyhydroxybenzoates, as well as block copolyether esters derived from hydroxyl-terminated polyethers; and also polyesters modified with polycarbonates or MBS.
19. Polyketones.
20. Polysulphones, polyether sulphones and polyether ketones.
21. Blends of the aforementioned polymers (polyblends), for example PP/EPDM, Polyamide/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPO/HIPS, PPO/PA 6.6 and copolymers, PA/HDPE, PA/PP, PA/PPO, PBT/PC/ABS or PBT/PET/PC.

Component a) is added to the substrate of component b) in an amount of about 0.001 to 5.0 weight%, preferably 0.01 to 2.0 weight% and most preferably 0.04 to 0.5 weight%.

A further embodiment of the invention relates to a process for imparting flame retardancy to a polymer substrate comprising polycarbonates or polycarbonate blends, which process comprises adding to said polymer substrate the salt of a selected aromatic sulphonic acid as defined above.

### Additional Components

The instant invention further pertains to a composition, which comprises, in addition to the components a) and b), as defined above, further additives selected from the group consisting of polymer stabilizers and additional flame-retardants, such as phosphorus containing flame-retardants, nitrogen containing flame-retardants, halogenated flame-retardants and inorganic flame-retardants.

The invention relates to a composition which additionally comprises as additional component so-called anti-dripping agents.

These anti-dripping agents reduce the melt flow of the polycarbonate composition and inhibit the formation of drops at high temperatures. Various references, such as U.S. Patent Specification No. 4,263,201*,* describe the addition of anti-dripping agents to polycarbonate flame retardant compositions.

Suitable additives that inhibit the formation of drops at high temperatures include glass fibers, polytetrafluoroethylene (PTFE), high temperature elastomers, carbon fibers, glass spheres and the like.

The addition of polysiloxanes of different structures has been proposed in various references; cf. U.S. Patent Specification Nos. 6,660,787*,* 6,727,302 or 6,730,720*.*

According to a specific embodiment of the invention a (poly)carbonate compound of the formula

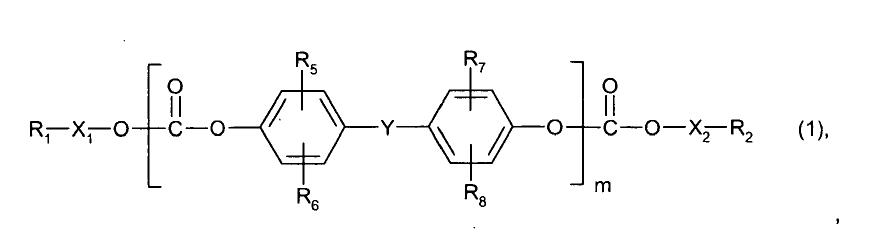

Wherein
R₁ and R₂ independently of one another represent an aliphatic group substituted by fluorine; X₁ and X₂ independently of one another represent the direct bond or C₁-C₁₂alkylene;
m represents a numeral from 1 to 1000;
R₅, R₆, R₇ and R₈ independently of one another represent hydrogen, C₁-C₁₂alkyl or C₃-C₁₂alkenyl; and
Y represents the direct bond or a bivalent group selected from the group consisting of -O-,

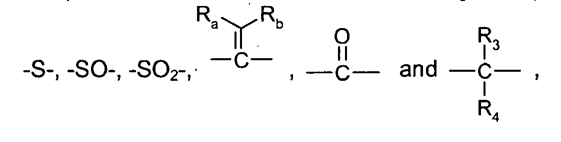

Wherein
Both Rₐ and R_{b} represent hydrogen or halogen; or
One of Rₐ and R_{b} represents hydrogen and the other one represents halogen;
R₃ and R₄, together with the carbon atom to which they are bonded, form a C₅-C₈-cycloalkylidene group with 1 to 3 C₁-C₄alkyl groups as optional substituents; or
R₃ and R₄ independently of one another represent hydrogen, an aliphatic group substituted by fluorine, C₁-C₁₂alkyl, C₁-C₁₂alkyl substituted by carboxy, C₂-C₁₂alkenyl, aryl, or the group of the partial formula

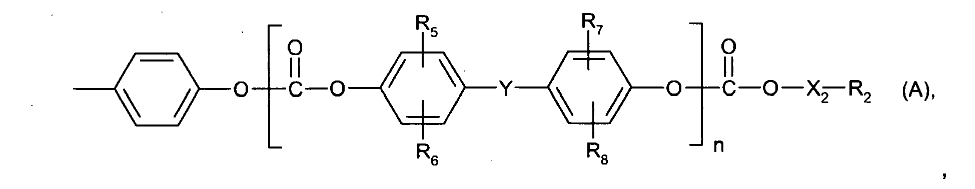

Wherein
n represents a numeral from 0-10 000; and
X₂, Y, R₂, R₅, R₆, R₇ and R₈ are as defined above.

In the (poly)carbonate compound (1), as defined above, the substituents are defined as follows:
R₁ and R₂ independently of one another represent an aliphatic group substituted by fluorine;
X₁ and X₂ independently of one another represent the direct bond or C₁-C₁₂alkylene;
m represents a numeral from 1 to 1000;
R₅, R₆, R₇ and R₈ independently of one another represent hydrogen, C₁-C₁₂alkyl or C₃-C₁₂alkenyl; and
Y represents the direct bond or a bivalent group selected from the group consisting of -O-, -S-, -SO-, -SO₂-, 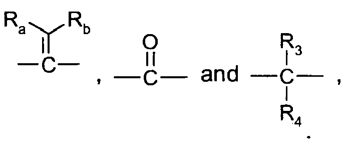 wherein

Both Rₐ and R_{b} represent hydrogen or halogen; or
One of Rₐ and R_{b} represents hydrogen and the other one represents halogen;
R₃ and R₄, together with the carbon atom to which they are bonded, form a C₅-C₈-cycloalkylidene group with 1 to 3 C₁-C₄alkyl groups as optional substituents; or
R₃ and R₄ independently of one another represent hydrogen, an aliphatic group substituted by fluorine, C₁-C₁₂alkyl, C₁-C₁₂alkyl substituted by carboxy, C₂-C₁₂alkenyl, aryl, or the group (A), as defined above, wherein n represents a numeral from 0-10 000 and X₂, Y, R₂, R₅, R₆, R₇ and R₈ are as defined above.

R₁ and R₂ defined as an aliphatic group substituted by fluorine is preferably a straight chain or branched or hydrocarbon group, which contains at least one fluoro atom and at least one hydrogen atom, for example fluoro-C₁-C₂₅alkyl, or is a perfluoroalkyl group of the partial formula

-(CF₂)ₚF (B),

wherein p is a numeral from 1 to 100.

Fluoro-C₁-C₂₅alkyl is for example, mono-, or difluoromethyl, 2-fluoroethyl, 3-fluoropropyl, 4-fluorobutyl, 5-fluoropentyl, 6-fluorohexyl, 7-fluoroheptyl, or pentafluorobutyl.

Perfluoroalkyl is a group (B) derived from the perfluoro alcohol F(CF₂)ₚ-OH wherein p is 1 to 50, for example trifluoromethyl (p = 1) or pentafluoroethyl (p = 2). Preferred perfluoroalkyl groups are derived form perfluoro alcohols wherein p is 5, 8, 9 or 11.

X₁ and X₂ defined as C₁-C₁₂alkylene is a branched or unbranched bivalent group, for example methylene, ethylene, propylene, trimethylene, tetramethylene, pentamethylene, hexamethylene, heptamethylene, octamethylene, decamethylene or dodecamethylene. One of the preferred definitions for X₁ and X₂ is C₁-C₈alkylene, for example C₂-C₈alkylene. An especially preferred definition for X₁ and X₂ is C₂-C₄alkylene, for example ethylene.

R₅, R₆, R₇ and R₈ defined as C₁-C₁₂alkyl is a straight chain or, where possible, branched radical, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, n-nonyl, n-decyl, n-undecyl, 1-methylundecyl or n-dodecyl. One of the preferred definitions is, for example, C₁-C₈alkyl, for example C₁-C₄alkyl, such as methyl.

R₅, R₆, R₇ and R₈ defined as C₃-C₁₂alkenyl is a straight chain or, where possible, branched radical, for example allyl, 2-butenyl, 3-butenyl, isobutenyl, n-2,4-pentadienyl, 3-methyl-2-butenyl, n-2-octenyl, n-2-dodecenyl or iso-dodecenyl.

Y defined as a bivalent group of the partial formula 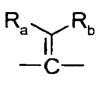 is preferably methylene (CH₂=, Rₐ and R_{b} = H). According to alternative embodiments, both Rₐ and R_{b} represent hydrogen or halogen, for example chlorine or bromine, or one of Rₐ and R_{b} represents hydrogen and the other one represents halogen.

R₃ and R₄ defined as a C₅-C₈-cycloalkylidene group with 1 to 3 C₁-C₄alkyl groups as optional substituents is, for example, cyclopentylidene, methylcyclopentylidene, dimethylcyclopentylidene, cyclohexylidene, methylcyclohexylidene, dimethylcyclohexylidene, trimethylcyclohexylidene, tert-butylcyclohexylidene, cycloheptylidene or cyclooctylidene. Preference is given to cyclohexylidene.

R₃ and R₄ defined as an aliphatic group substituted by fluorine is for example fluoro-C₁-C₂₅alkyl, as defined above, or is the above-mentioned perfluoroalkyl group (B), wherein p is 1 to 50.

R₃ and R₄ defined as C₁-C₁₂alkyl is as defined above with regard to R₅, R₆, R₇ and R₈.

R₃ and R₄ defined as C₁-C₁₂alkyl substituted by carboxy is, for example, carboxymethyl or 1- or 2-carboxyethyl.

R₃ and R₄ defined as aryl is preferably phenyl or 1- or 2-naphthyl.

In the group (A) the index n represents a numeral from 0-10 000 and X₂, Y, R₂, R₅, R₆, R₇ and R₈ are as defined above.

Of particular interest are (poly)carbonate compounds (1), wherein
R₁ and R₂ independently of one another represent an aliphatic group substituted by fluorine;
X₁ and X₂ independently of one another represent C₁-C₁₂alkylene;
m represents a numeral from 1 to 1 000;
R₅, R₆, R₇ and R₈ represent hydrogen;
Y represents the bivalent group 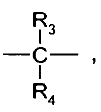 wherein independently of one another R₃ and R₄
represent hydrogen, -CF₃, C₁-C₁₂alkyl, phenyl or the group (A), wherein n represents a numeral from 0 to 10 000 and X₂, Y, R₂, R₅, R₆, R₇ and R₈ are as defined above or R₃ and R₄, together with the carbon atom to which they are bonded, form the cyclohexylidene group with 1 to 3 C₁-C₄alkyl groups as optional substituents.

Of particular interest are also (poly)carbonate compounds (1), wherein R₁ and R₂ independently of one another represent groups (B), wherein p is a numeral from 1 to 50.

Of special interest are (poly)carbonate compounds (1), wherein p is a numeral from 4 to 15.

Of very special interest are (poly)carbonate compounds (1), wherein

R₁ and R₂ independently of one another represent groups (B), wherein p is a numeral from 1 to 50;
X₁ and X₂ independently of one another represent C₂-C₈alkylene;
m represents a numeral from 1 to 1 000;
R₅, R₆, R₇ and R₈ represent hydrogen; and
Y represents the bivalent group 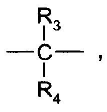 wherein

R₃ represents hydrogen, -CF₃, C₁-C₁₂alkyl, phenyl or the group (A), wherein the numeral n represents a numeral from 0 to 10 000 and X₂, Y, R₂, R₅, R₆, R₇ and R₈ are as defined above or R₃ and R₄, together with the carbon atom to which they are bonded, form the cyclohexylidene group with 1 to 3 C₁-C₄alkyl groups as optional substituents.

Of high interest are (poly)carbonate compounds (1), wherein R₃ and R₄ independently of one another represent hydrogen or C₁-C₄alkyl; or R₃ and R₄, together with the carbon atom to which they are bonded, form the cyclohexylidene group.

Particularly preferred are also (poly)carbonate compounds (1), wherein m is a numeral from 1 to 50, and n is a numeral from 0 to 50.

The (poly)carbonate compounds (1) are prepared by known methods. A fluoro alcohol is treated with bis(2,4-dinitrophenyl)carbonate (DNPC) to give the 2,4-dinitrophenyl carbonate of the fluoro alcohol *in situ.* This derivative can be isolated and treated separately, for example by hydroxy terminated bisphenol A oligomers of various molecular weights.

Brunelle et al., Macromolecules 1991, 24, 3035-3044*,* discloses the use of bis(2,4-dinitrophenyl)carbonate for preparation of dimer and cyclic oligomers of bisphenol A. The coupling reactions can also be carried out by carbonate linkage forming reagents, such as phosgene or carbonyl diimidazole (CDI).

Preferred fluoro alcohols are, for example, so-called fluorotelomer alcohols. These are, for example, commercially available from DuPont or Aldrich as Zonyl® BA-L.

Preferred bisphenol starting materials are, for example, bisphenol A and the compounds of the formulae:

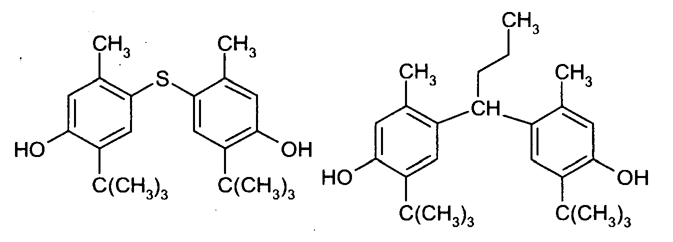

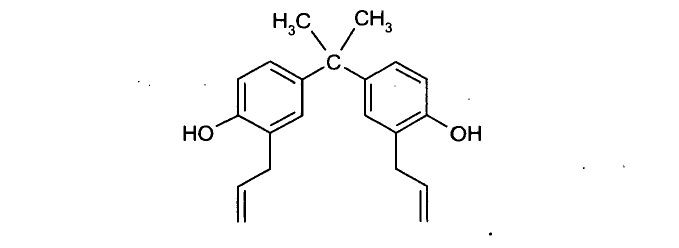

According to an alternative embodiment a (poly)carbonate compound of the formula 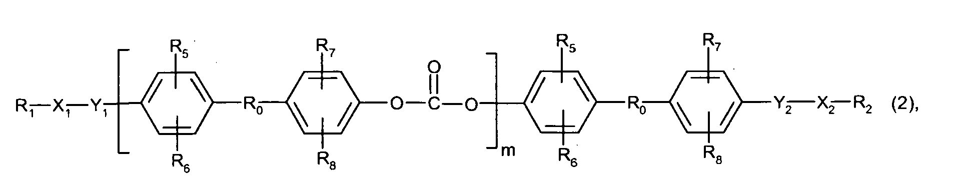 is added, wherein
R₀ represents the direct bond or a bivalent group selected from the group consisting 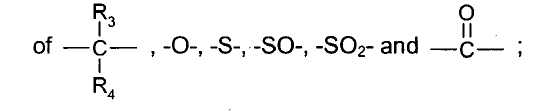 R₁ and R₂ independently of one another represent a silicon containing group;
R₃ and R₄ independently of one another represent hydrogen, an aliphatic group substituted by fluorine, a silicon containing group, C₁-C₁₂alkyl, C₁-C₁₂alkyl substituted by carboxy, C₂-C₁₂alkenyl, aryl, or a group of the partial formula 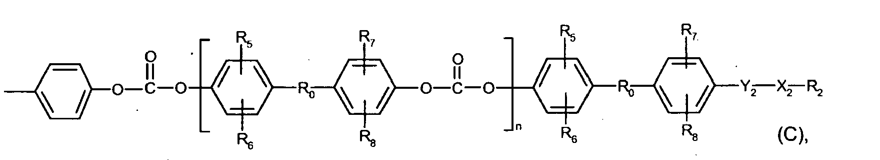 or R₃ and R₄, together with the carbon atom to which they are bonded represent C₅-C₈-cycloalkylidene,or C₅-C₈-cycloalkylidene that is substituted by from 1 to 3 C₁-C₄alkyl groups;
R₅, R₆, R₇ and R₈ independently of one another represent hydrogen, C₁-Cₗ₂alkyl or C₃-C₁₂alkenyl;
X₁ and X₂ independently of one another represent the direct bond, C₁-C₁₂alkylene or C₄-C₂₅alkylene interrupted by -O-;
Y₁ and Y₂ independently of one another represent the direct bond or a bivalent group selected from the group consisting of -O-, 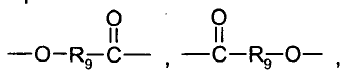 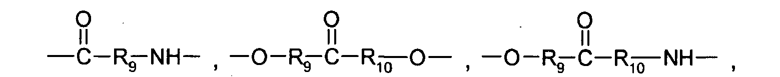 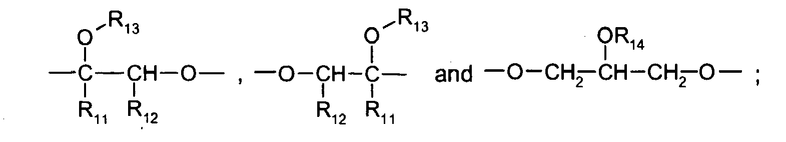 R₉ and R₁₀ independently of one another represent the direct bond or C₁-C₄alkylene;
R₁₁, R₁₂ and R₁₃ independently of one another represent hydrogen, C₁-C₁₂alkyl or C₃-C₁₂alkenyl;
R₁₄ represents hydrogen, C₁-C₁₂alkyl or a silicon containing group,
m represents a numeral from 0 to 10 000; and
n represents a numeral from 0 to 10 000.

In the (poly)carbonate compound (2), as defined above, the substituents are defined as follows:
R₀ represents the direct bond or a bivalent group selected from the group consisting of 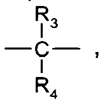 -O-, -S-, -SO-, -SO₂- and 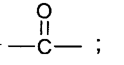 R₁ and R₂ independently of one another represent a silicon containing group;
R₃ and R₄ independently of one another represent hydrogen, an aliphatic group substituted by fluorine, a silicon containing group, C₁-C₁₂alkyl, C₁-C₁₂alkyl substituted by carboxy, C₂-C₁₂alkenyl, aryl, or a group of the partial formula 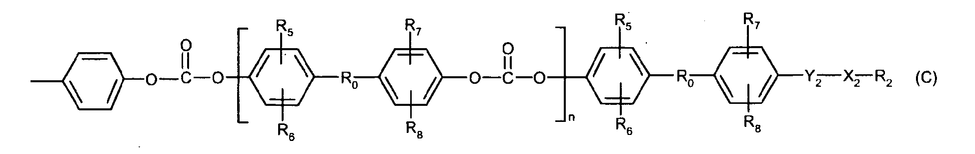 or R₃ and R₄, together with the carbon atom to which they are bonded represent C₅-C₈-cycloalkylidene or C₅-C₈-cycloalkylidene that is substituted by 1 to 3 C₁-C₄alkyl groups;
R₅, R₆, R₇ and R₈ independently of one another represent hydrogen, C₁-C₁₂alkyl or C₃-C₁₂alkenyl;
X₁ and X₂ independently of one another represent the direct bond, C₁-C₁₂alkylene or C₄-C₂₅alkylene interrupted by -O-;
Y₁ and Y₂ independently of one another represent the direct bond or a bivalent group selected from the group consisting of -O-, 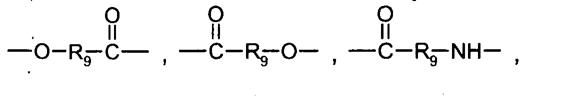 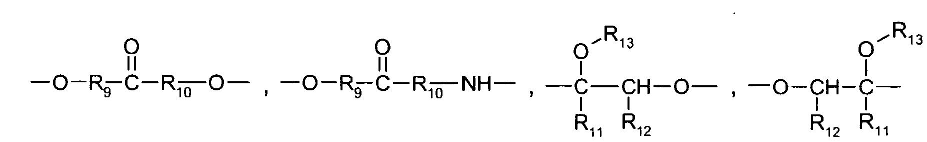 and 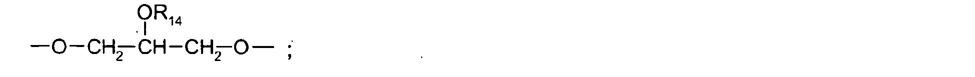 R₉ and R₁₀ independently of one another represent the direct bond or C₁-C₄alkylene;
R₁₁, R₁₂ and R₁₃ independently of one another represent hydrogen, C₁-C₁₂alkyl or C₃-C₁₂alkenyl;
R₁₄ represents hydrogen, C₁-C₁₂alkyl or a silicon containing group;
m represents a numeral from 0 to 10 000; and
n represents a numeral from 0 to 10 000.

A silicon containing group preferably represents a group of the partial formula 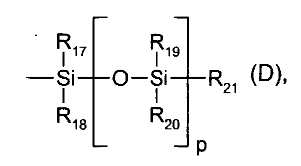 wherein
R₁₇, R₁₈, R₁₉ and R₂₀ independently of one another represent C₁-C₁₂alkyl, C₁-C₁₂alkyl substituted with hydroxy or amino; hydroxyC₄-C₁₂alkyl interrupted with -O-; or represents a group of the partial formula 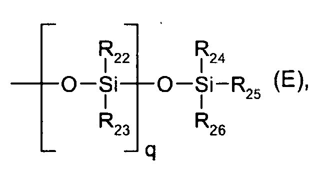 wherein
R₂₁ represents C₁-C₁₂alkyl or a group of the partial formula 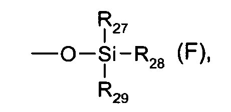 R₂₂, R₂₃, R_{24,} R₂₅, R₂₆, R₂₇, R₂₈ and R₂₉ independently of one another represent C₁-C₁₂alkyl or C₁-C₁₂-alkyl substituted with hydroxy or amino;
p represents 0 to 200; and
q represent 0 to 200.

Of special interest as a silicon containing group is a group of the partial formula (D), wherein R₁₇, R₁₈, R₁₉ and R₂₀ independently of one another represent methyl or a group of the partial formula 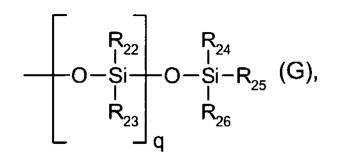 R₂₁ represents methyl or a group of the partial formula 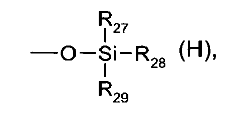 R₂₂, R₂₃, R₂₄, R₂₅, R₂₆, R₂₇, R₂₈ and R₂₉ are methyl; and
p and q independently of one another represent 0 to 100.

Of particular interest are (poly)carbonate compounds (2), wherein
R₀ represents the bivalent group 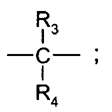 R₁ and R₂ independently of one another represent a silicon containing group;
R₃ and R₄ independently of one another represent hydrogen, trifluoromethyl, a silicon containing group, C₁-C₁₂alkyl, phenyl or the group (C); or
R₃ and R₄, together with the carbon atom to which they are bonded represent C₅-C₈-cycloalkylidene or C₅-C₈-cycloalkylidene that is substituted by 1 to 3 C₁-C₄alkyl groups;
R₅, R₆, R₇ and R₈ are hydrogen;
X₁ and X₂ independently of one another represent C₁-C₁₂alkylene or C₄-C₂₅alkylene interrupted by -O-;
Y₁ and Y₂ independently of one another represent the direct bond or a bivalent group selected from the group consisting of -O-, 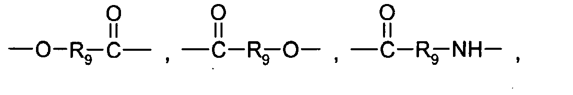 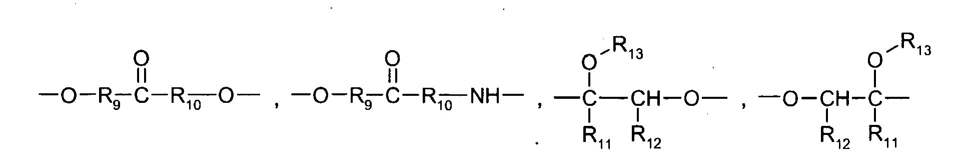 and 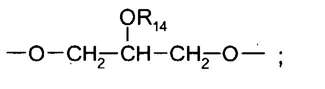 R₉ and R₁₀ independently of one another represent the direct bond or methylene;
R₁₁, R₁₂ and R₁₃ independently of one another represent hydrogen, C₁-C₄alkyl or C₃-C₄alkenyl;
R₁₄ represents hydrogen or C₁-C₁₂alkyl;
m represents 0 to 10 000; and
n represents 0 to 10 000.

Of very special interest are (poly)carbonate compounds (2), wherein
R₀ represents the bivalent group 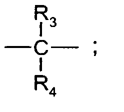 R₃ represents hydrogen, -CF₃, C₁-C₁₂alkyl, phenyl or the group (C);
R₄ represents -CF₃, C₁-C₁₂alkyl or phenyl; or
R₃ and R₄, together with the carbon atom to which they are bonded, form a C₅-C₈-cycloalkylidene group or C₅-C₈-cycloalkylidene that is substituted by 1 to 3 C₁-C₄alkyl groups;
R₅, R₆, R₇ and R₈ represent hydrogen;
X₁ and X₂ are each independently of the one another represent C₁-C₁₂alkylene or C₄-C₂₅alkylene interrupted by -O-;
Y₁ and Y₂ independently of one another represent the direct bond or a bivalent group selected from the group consisting of -O-, 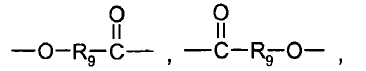 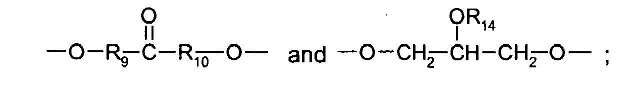 R₉ and R₁₀ independently of one another represent the direct bond or methylene;
R₁₄ represents hydrogen or C₁-C₁₂alkyl;
m represents 0 to 10 000; and
n represents 0 to 10 000.

Of interest are also (poly)carbonate compounds (2), wherein
R₃ and R₄ independently of one another represent hydrogen or C₁-C₄alkyl; or

R₃ and R₄, together with the carbon atom to which they are bonded, form the cyclohexylidene group.

Preferred are (poly)carbonate compounds (2), wherein X₁ and X₂ independently of one another represent C₂-C₈alkylene or C₄-C₂₅alkylene interrupted with -O-.

Also preferred are (poly)carbonate compounds (2), wherein m represents 0 to 100, and n represents 0 to 100.

Of very special interest are (poly)carbonate compounds (2), wherein
R₀ represents the bivalent group 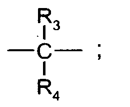 R₃ and R₄ independently of one another represent C₁-C₄alkyl; or
R₃ and R₄, together with the carbon atom to which they are bonded, form the cyclohexylidene group;
R₅, R₆, R₇ and R₈ represent hydrogen;
X₁ and X₂ independently of one another represent C₂-C₄alkylene or C₄-C₂₅alkylene interrupted with -O-;
Y₁ and Y₂ independently of one another represent the direct bond or a bivalent group selected from the group consisting of -O-, 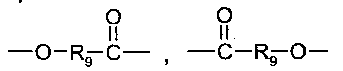 and 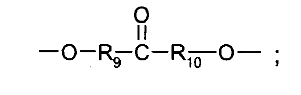 R₉ and R₁₀ independently of one another represent the direct bond or methylene;
m represents 0 to 100, and
n represents 0 to 100.

In a (poly)carbonate compound (2) C₁-C₁₂alkyl is a straight chain or, where possible, branched alkyl group, which is the same as defined above with regard to (poly)carbonate compounds (1).

R₃ and R₄ defined as C₁-C₁₂alkyl substituted by carboxy is preferably carboxymethyl or 1- or 2-carboxyethyl.

R₃ and R₄ defined as aryl preferably represent phenyl or phenyl substituted by 1-3 C₁-C₄alkyl groups, e.g. methyl.

R₃, R₄, R₅, R₆, R₇ and R₈ defined as C₂-C₁₂alkenyl represent a straight chain or, where possible, branched alkenyl group, which is the same as defined above with regard to (poly)carbonate compounds (1).

R₃ and R₄ defined as C₅-C₈-cycloalkylidene or C₅-C₈-cycloalkylidene that is substituted by from 1 to 3 C₁-C₄alkyl groups are as defined above with regard to (poly)carbonate compounds (1).

X₁, and X₂, defined as C₁-C₁₂alkylene and R₉ and R₁₀ defined as C₁-C₄alkylene represent straight chain or, where possible, branched alkylene groups as defined above with regard to (poly)carbonate compounds (1).

X₁, and X₂, defined as C₄-C₂₅alkylene interrupted with -O- is straight chain or, where possible, branched, for example -CH₂CH₂-O-CH₂CH₂-, -CH₂CH₂CH₂-O-CH₂CH₂-, -CH₂CH₂CH₂-O-CH₂CH₂CH₂- or -CH₂CH₂-O-CH₂CH₂-O-CH₂CH₂-.

C₁-C₁₂Alkyl substituted with hydroxy or amino is, for example, hydroxyethyl, 1- or 2-hydroxyethyl, aminomethyl or 1- or 2-aminoethyl.

Hydroxy-C₄-C₁₂alkyl interrupted with -O- is for example -CH₂CH₂-O-CH₂CH₂OH or -CH₂CH₂-O-CH₂CH₂-O-CH₂CH₂OH.

A fluorine containing group is a branched or unbranched radical, which contains at least one fluoro atom, for example fluoro-C₁-C₂₅alkyl; or is the group (B), wherein p is 1 to 50.

Fluoro-C₁-C₂₅alkyl is for example perfluoroalkyl, fluoromethyl, 2-fluoroethyl, 3-fluoropropyl, 4-fluorobutyl, 5-fluoropentyl, 6-fluorohexyl, 7-fluoroheptyl, difluoromethyl, trifluoromethyl, pentafluoroethyl, pentafluorobutyl.

The (poly)carbonate compounds (2) are obtainable by known methods. A silicon alcohol is treated with bis(2,4-dinitrophenyl)carbonate (DNPC) to give the 2,4-dinitrophenyl carbonate of the silicon alcohol *in situ.* This derivative can be isolated and treated separately, for example by hydroxy terminated bisphenol A oligomers of various molecular weights. Brunelle et al., Macromolecules 1991, 24, 3035-3044*,* discloses the use of bis(2,4-dinitro-phenyl)carbonate for preparation of dimer and cyclic oligomers of bisphenol A. The coupling reactions can also be carried out by carbonate linkage forming reagents, such as phosgene or carbonyl diimidazole (CDI).

Especially preferred silicon containing groups are derived from mono hydroxypolysiloxanes, wherein p=10; polysiloxanes, wherein p=64; polyalkylene oxides modified heptamethyltrisiloxanes; or 3-(polyoxyethylene)propylheptamethyltrisiloxane. Representative structural formulae are shown below:

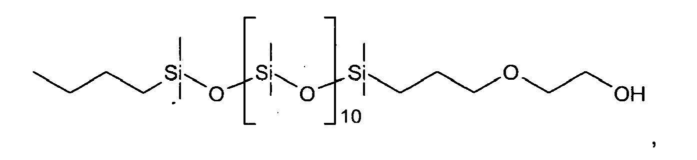

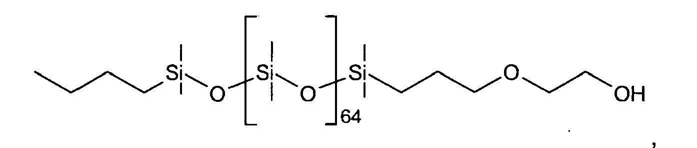

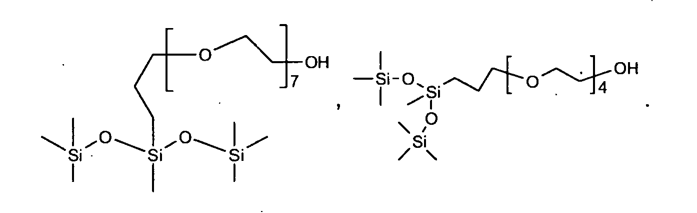

Preferred bisphenol starting materials are the same as the ones mentioned above with regard to the preparation of the (poly)carbonate compounds (1).

(poly)carbonate compounds (1) or (2) are added to the substrate of component b) in an amount of about 0.01 to 5.0 weight% and preferably 0.25 to 1.0 weight%. The addition of mixtures of (poly)carbonate compounds (1) or (2) is suggested.

According to another embodiment the invention relates to a composition which additionally comprises further additives in selected from the group consisting of polymer stabilizers and additional flame-retardants.

Stabilizers are preferably halogen-free and selected from nitroxyl stabilizers, nitrone stabilizers, amine oxide stabilizers, benzofuranone stabilizers, phosphite and phosphonite stabilizers, quinone methide stabilizers and monoacrylate esters of 2,2'-alkylidenebisphenol stabilizers.

Additional flame-retardants are known components, items of commerce or can be obtained by known methods.

Representative phosphorus containing flame-retardants, in addition to the ones defined above with regard to component b), are for example:

Tetraphenyl resorcinol diphosphite (FYROLFLEX^{®} RDP, Akzo Nobel), tetrakis(hydroxymethyl)phosphonium sulphide, triphenyl phosphate, diethyl-N,N-bis(2-hydroxyethyl)-aminomethyl phosphonate, hydroxyalkyl esters of phosphorus acids, ammonium polyphosphate (APP) or (HOSTAFLAM^{®} AP750), resorcinol diphosphate oligomer (RDP), phosphazene flame-retardants and ethylenediamine diphosphate (EDAP).

Nitrogen containing flame-retardants are, for example, isocyanurate flame-retardants, such as polyisocyanurate, esters of isocyanuric acid or isocyanurates. Representative examples are hydroxyalkyl isocyanurates, such as tris-(2-hydroxyethyt)isocyanurate, tris(hydroxymethyl)isocyanurate, tris(3-hydroxy-n-proyl)isocyanurate or triglycidyl isocyanurate.

Nitrogen containing flame-retardants include melamine-based flame-retardants. Representative examples are: melamine cyanurate, melamine borate, melamine phosphates, melamine polyphosphate, melamine pyrophosphate, melamine ammonium polyphosphate and melamine ammonium pyrophosphate.

Further examples are: benzoguanamine, tris(hydroxyethyl) isocyanurate, allantoin, glycoluril, melamine cyanurate, melamine phosphate, dimelamine phosphate, melamine pyrophosphate, urea cyanurate, melamine polyphosphate, melamine borate, ammonium polyphosphate, melamine ammonium polyphosphate or melamine ammonium pyrophosphate, a condensation product of melamine from the series melem, melam, melon and/or a higher condensed compound or a reaction product of melamine with phosphoric acid and/or a reaction product of condensation products of melamine with phosphoric acid or a mixture thereof.

Special emphasis should be given to: dimelamine pyrophosphate, melamine polyphosphate, melem polyphosphate, melam polyphosphate, and/or a mixed polysalt of such a type, more especially melamine polyphosphate.

Representative organohalogen flame-retardants are, for example:
Polybrominated diphenyl oxide (DE-60F, Great Lakes Corp.), decabromodiphenyl oxide (DBDPO; SAYTEX^{®} 102E), tris[3-bromo-2,2-bis(bromomethyl)propyl] phosphate (PB 370^{®}, FMC Corp.), tris(2,3-dibromopropyl)phosphate, tris(2,3-dichloropropyl)phosphate, chlorendic acid, tetrachlorophthalic acid, tetrabromophthalic acid, poly-β-chloroethyl triphosphonate mixture, tetrabromobisphenol A bis(2,3-dibromopropyl ether) (PE68), brominated epoxy resin, ethylene-bis(tetrabromophthalimide) (SAYTEX^{®} BT-93), bis(hexachlorocyclopentadieno)-cyclooctane (DECLORANE PLUS^{®}), chlorinated paraffins, octabromodiphenyl ether, hexachlorocyclopentadiene derivatives, 1,2-bis(tribromophenoxy)ethane (FF680), tetrabromobisphenol A (SAYTEX^{®} RB100), ethylene bis-(dibromo-norbornanedicarboximide) (SAYTEX^{®} BN-451), bis-(hexachlorocycloentadeno) cyclooctane, PTFE, tris-(2,3-dibromopropyl)-isocyanurate, and ethylene-bis-tetrabromophthalimide.

The flame-retardant mentioned above routinely combined with an inorganic oxide synergist. Most common for this use are zinc or antimony oxides, e.g. Sb₂O₃ or Sb₂O₅. Boron compounds are suitable, too.

The above-mentioned additional flame-retardant classes are advantageously contained in the composition of the invention in an amount from about 0.5% to about 45.0% by weight of the organic polymer substrate; for instance about 1.0% to about 40.0%; for example about 5.0% to about 35.0% by weight of the polymer.

As mentioned above, the composition according to the invention may additionally contain one or more conventional additives, for example selected from pigments, dyes, plasticizers, antioxidants, thixotropic agents, levelling assistants, basic co-stabilizers, metal passivators, metal oxides, organophosphorus compounds, further light stabilizers and mixtures thereof, especially pigments, phenolic antioxidants, calcium stearate, zinc stearate, UV absorbers of the 2-hydroxy-benzophenone, 2-(2'-hydroxyphenyl)benzotriazole and/or 2-(2-hydroxyphenyl)-1,3,5-triazine groups. More specific examples are the following components:
1. Antioxidants
   1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-tert-butyl-4-methoxymethylphenol, nonylphenols which are linear or branched in the side chains, for example 2,6-di-nonyl-4-methylphenol, 2,4-dimethyl-6-(1'-methylundec-1'-yl)phenol, 2,4-dimethyl-6-(1'-methylheptadec-1'-yl)phenol, 2,4-dimethyl-6-(1'-methyltridec-1'-yl)phenol and mixtures thereof.
   1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dode-cylthiomethyl-4-nonylphenol.
   1.3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis(3,5-di-tert-butyl-4-hydroxyphenyl) adipate.
   1.4. Tocopherols, for example α-, β-, γ-, δ-tocopherol and mixtures thereof (vitamin E).
   1.5. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thio-bis(6-tert-butyl-2-methylphenol), 4,4'-thiobis(3,6-di-sec-amylphenol), 4,4'-bis(2,6-dimethyl-4-hydroxyphenyl)disulphide.
   1.6. Alkylidenebisphenols, for example 2,2'-methylenebis(6-tert-butyl-4-methylphenol), 2,2'-methylenebis(6-tert-butyl-4-ethylphenol), 2,2'-methylenebis[4-methyl-6-(α-methylcyclohexyl)phenol], 2,2'-methylenebis(4-methyl-6-cyclohexylphenol), 2,2'-methylenebis(6-nonyl-4-methylphenol), 2,2'-methyleriebis(4,6-di-tert-butylphenol), 2,2'-ethylidenebis(4,6-di-tert-butylphenol), 2,2'-ethylidenebis(6-tert-butyl-4-isobutylphenol), 2,2'-methylenebis[6-(α-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3'-tert-butyl-4'-hydroxyphenyl)butyrate], bis(3-tert-butyl-4-hydroxy-5-methyl-phenyl)dicyclopentadiene, bis[2-(3'-tert-butyl-2'-hydroxy-5'-methylbenzyl)-6-tert-butyl-4-methylphenyl]terephthalate, 1,1-bis-(3,5-dimethyl-2-hydroxyphenyl)butane, 2,2-bis(3,5-di-tert-butyl-4-hydroxyphenyl)propane, 2,2-bis-(5-tert-butyl-4-hydroxy2-methylphenyl)-4-n-dodecylmercaptobutane, 1,1,5,5-tetra(5-tert-butyl-4-hydroxy-2-methylphenyl)pentane.
   1.7. O-, N- and S-benzyl compounds, for example 3,5,3',5'-tetra-tert-butyl-4,4'-dihydroxy-dibenzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)dithioterephthalate, bis(3,5-di-tert-butyl-4-hydroxybenzyl)sulphide, isooctyl-3,5-di-tert-butyl-4-hydroxybenzylmercaptoacetate.
   1.8. Hydroxybenzylated malonates, for example dioctadecyl-2,2-bis(3,5-di-tert-butyl-2-hydroxybenzyl)malonate, di-octadecyl-2-(3-tert-butyl-4-hydroxy-5-methylbenzyl)malonate, di-dodecylmercaptoethyl-2,2-bis(3,5-di-tert-butyl-4-hydroxybenzyl)malonate, bis[4-(1,1,3,3-tetramethylbutyl)phenyl]-2,2-bis(3,5-di-tert-butyl-4-hydroxybenzyl)malonate.
   1.9. Aromatic hydroxybenzyl compounds, for example 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene, 2,4,6-tris(3,5-di-tert-butyl-4-hydroxybenzyl)phenol.
   1.10. Triazine compounds, for example 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris(3,5-di-tert-butyl-4-hydroxyphenoxy)-1,2,3-triazine, 1,3,5-tris(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris(3,5-di-tert-butyl-4-hydroxyphenylethyl)-1,3,5-triazine, 1,3,5-tris(3,5-di-tert-butyl-4-hydroxyphenylpropionyl)-hexahydro-1,3,5-triazine, 1,3,5-tris(3,5-dicyclohexyl-4-hydroxybenzyl)isocyanurate.
   1.11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl3,5-di-tert-butyl-4-hydroxybenzylphosphonate, dioctadecyl-5-tert-butyl-4-hydroxy-3-methylbenzyl-phosphonate, the calcium salt of the monoethyl ester of 3,5-di-tert-butyl-4-hydroxy-benzylphosphonic acid.
   1.12. Acylaminophenols, for example 4-hydroxylauranilide, 4-hydroxystearanilide, octyl N-(3,5-di-tert-butyl-4-hydroxyphenyl)carbamate.
   1.13. Esters of β-(3,5-di-tert-butyl-4-hydroxyphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane, such as commercially available products like Irganox® 1076.
   1.14. Esters of β-(5-tert-butyl-4-hydroxy-3-methylphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)-isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane; 3,9-bis[2-{3-(3-tert-butyl-4-hydroxy-5-methylphenyl)propionyloxy}-1,1-dimethylethyl]-2,4,8,10-tetraoxaspiro[5.5]undecane.
   1.15. Esters of β-(3,5-dicyclohexyl-4-hydroxyphenyl)propionic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo-[2.2.2]octane.
   1.16. Esters of 3,5-di-tert-butyl-4-hydroxyphenyl acetic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.17. Amides of β-(3,5-di-tert-butyl-4-hydroxyphenyl)propionic acid e.g. N,N'-bis(3,5-di-tert-butyl-4-hydroxyphenylpropionyl)hexamethylenediamide, N,N'-bis(3,5-di-tert-butyl-4-hydroxyphenylpropionyl)trimethylenediamide, N,N'-bis(3,5-di-tert-butyl-4-hydroxyphenylpropionyl)hydrazide, N,N'-bis[2-(3-[3,5-di-tert-butyl-4-hydroxyphenyl]propionyloxy)ethyl]oxamide (Naugard^{®}XL-1, supplied by Uniroyal).
   1.18. Ascorbic acid (vitamin C)
2. Light stabilisers
   2.1. 2-(2'-Hydroxyphenyl)benzotriazoles, for example 2-(2'-hydroxy-5'-methylphenyl)benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chlorobenzotriazole, 2-(3'-tert-butyl-2'-hydroxy-5'-methylphenyl)-5-chlorobenzotriazole, 2-(3'-sec-butyl-5'-tert-butyl-2'-hydroxyphenyl)benzotriazole, 2-(2'-hydroxy-4'-octyloxyphenyl)benzotriazole, 2-(3',5'-di-tert-amyl-2'-hydroxyphenyl)benzotriazole, 2-(3',5'-bis(α,α-dimethylbenzyl)-2'-hydroxyphenyl)benzotriazole, 2-(3'-tert-butyl-2'-hydroxy-5'-(2-octyloxycarbonylethyl)phenyl)-5-chlorobenzotriazole, 2-(3'-tert-butyl-5'-[2-(2-ethylhexyloxy)carbonylethyl]-2'-hydroxyphenyl)-5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenylbenzotriazole, 2,2'-methylenebis[4-(1,1,3,3-tetramethylbutyl)-6-benzotriazole-2-ylphenol]; the transesterification product of 2-[3'-tert-butyl-5'-(2-methoxycarbonylethyl)-2'-hydroxyphenyl]-2H-benzotriazole with polyethylene glycol 300; [R-CH₂CH₂-COO-CH₂CH₂⁆₂ ,
      where R = 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)phenyl]benzotriazole; 2-[2'-hydroxy-3'-(1,1,3,3-tetramethylbutyl)-5'-(α,α-dimethylbenzyl)phenyl]benzotriazole, such as commercially available light stabilisers from the Tinuvin® series, such as TINUVIN 234, 326, 329, 350, 360 or TINUVIN 1577.
   2.2. 2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives.
   2.3. Esters of substituted and unsubstituted benzoic acids, for example 4-tert-butylphenyl salicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.
   2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline.
   2.5. Nickel compounds, for example nickel complexes of 2,2'-thiobis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the monoalkyl esters, e.g. the methyl or ethyl ester, of 4-hydroxy-3,5-di-tert-butylbenzylphosphonic acid, nickel complexes of ketoximes, e.g. of 2-hydroxy-4-methylphenylundecylketoxime, nickel complexes of 1-phenyl-4-lauroyl-5-hydroxypyrazole, with or without additional ligands.
   2.6. Oxamides, for example 4,4'-dioctyloxyoxanilide, 2,2'-diethoxyoxanilide, 2,2'-dioctyloxy-5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxy-disubstituted oxanilides and mixtures of o- and p-ethoxy-disubstituted oxanilides.
   2.7. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropyloxy)phenyl]-4,6-bis(2,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3-dodecyloxypropoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxypropoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxypropyloxy]-phenyl}-4,6-bis(2,4-dimethylphenyl)-1,3,5-triazine.
3. Metal deactivators, for example N,N'-diphenyloxamide, N-salicylal-N'-salicyloyl hydrazine, N,N'-bis(salicyloyl)hydrazine, N,N'-bis(3,5-di-tert-butyl-4-hydroxyphenylpropionyl)hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl dihydrazide, N,N'-bis(salicyloyl)thiopropionyl dihydrazide.
4. Further phosphites and phosphonites, for example triphenyl phosphite, diphenylalkyl phosphites, phenyldialkyl phosphites, tris(nonylphenyl) phosphite, trilauryl phosphite, trioctadecyl phosphite, distearylpentaerythritol diphosphite, tris(2,4-di-tert-butylphenyl) phosphite, diisodecyl pentaerythritol diphosphite, bis(2,4-di-tert-butylphenyl)pentaerythritol diphosphite, bis(2,4-di-cumylphenyl)pentaerythritol diphosphite, bis(2,6-di-tert-butyl-4-methylphenyl)pentaerythritol diphosphite, diisodecyloxypentaerythritol diphosphite, bis(2,4-di-tert-butyl-6-methylphenyl)pentaerythritol diphosphite, bis(2,4,6-tris(tert-butylphenyl)pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocin, bis(2,4-di-tert-butyl-6-methylphenyl)methyl phosphite, bis(2,4-di-tert-butyl-6-methylphenyl)ethyl phosphite, 6-fluoro-2,4,8,10-tetra-tert-butyl-12-methyl-dibenz[d,g]-1,3,2-dioxaphosphocin, 2,2',2"-nitrilo[triethyltris(3,3',5,5'-tetra-tert-butyl-1,1'-biphenyl-2,2'-diyl)phosphite], 2-ethylhexyl(3,3',5,5'-tetra-tert-butyl-1,1'-biphenyl-2,2'-diyl)phosphite, 5-butyl-5-ethyl-2-(2,4,6-tri-tert-butylphenoxy)-1,3,2-dioxaphosphirane.
   The following phosphites are especially preferred:

   Tris(2,4-di-tert-butylphenyl) phosphite (Irgafos^{®}168, Ciba Specialty Chemicals), tris(nonylphenyl) phosphite, 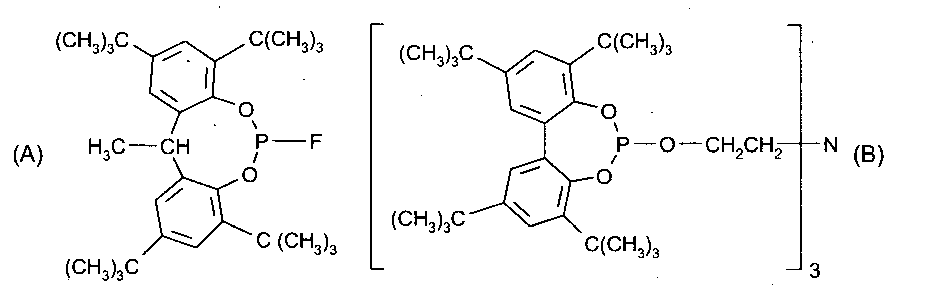 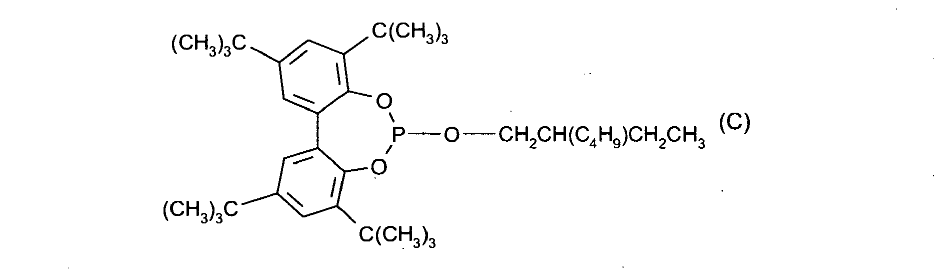 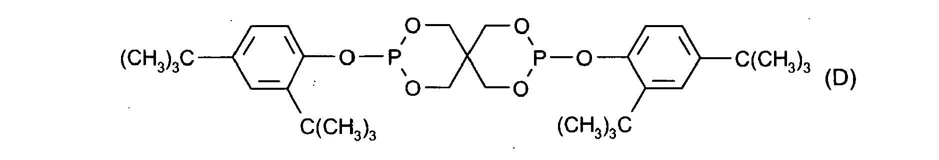 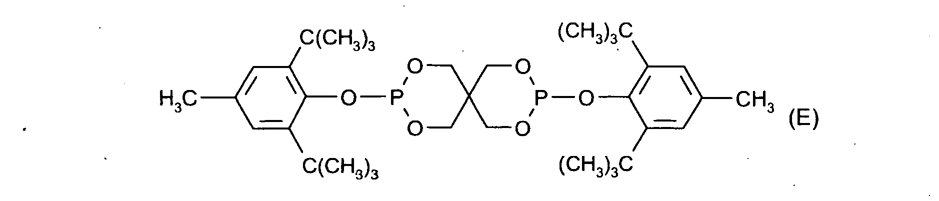 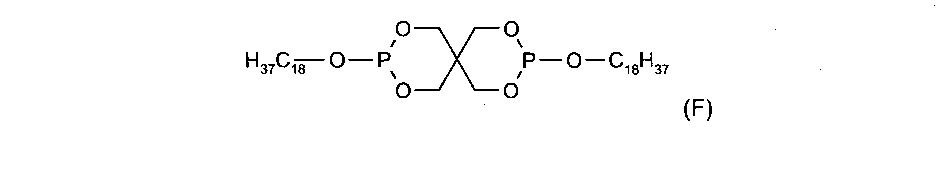 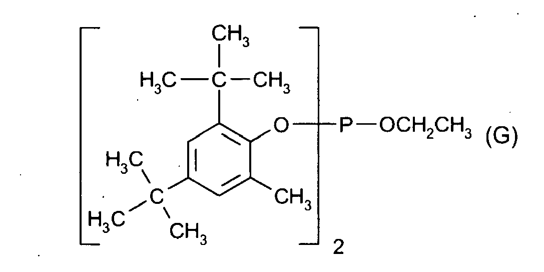
5. Further nitrones, for example N-benzyl-alpha-phenylnitrone, N-ethyl-alpha-methylnitrone, N-octyl-alpha-heptylnitrone, N-lauryl-alpha-undecylnitrone, N-tetradecyl-alpha-tridecylnitrone, N-hexadecyl-alpha-pentadecylnitrone, N-octadecyl-alpha-heptadecylnitrone, N-hexadecyl-alpha-heptadecylnitrone, N-ocatadecyl-alpha-pentadecylnitrone, N-heptadecyl-alpha-heptadecylnitrone, N-octadecyl-alpha-hexadecylnitrone, nitrone derived from N,N-dialkylhydroxylamine derived from hydrogenated tallow amine.
6. Thiosynergists, for example dilauryl thiodipropionate or distearyl thiodipropionate.
7. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulphide, pentaerythritol tetrakis(β-dodecylmercapto)propionate.
8. Polyamide stabilisers, for example copper salts in combination with iodides and/or phosphorus compounds and salts of divalent manganese.
9. Basic co-stabilisers, for example melamine, polyvinylpyrrolidone, dicyandiamide, triallyl cyanurate, urea derivatives, polyamides, polyurethanes, alkali metal salts and alkaline earth metal salts of higher fatty acids, for example calcium stearate, zinc stearate, magnesium behenate, magnesium stearate, sodium ricinoleate and potassium palmitate, antimony pyrocatecholate or zinc pyrocatecholate.
10. Nucleating agents, for example inorganic substances, such as talcum, metal oxides, such as titanium dioxide or magnesium oxide, phosphates, carbonates or sulphates of, preferably, alkaline earth metals; organic compounds, such as mono- or polycarboxylic acids and the salts thereof, e.g. 4-tert-butylbenzoic acid, adipic acid, diphenylacetic acid, sodium succinate or sodium benzoate; polymeric compounds, such as ionic copolymers (ionomers). Especially preferred are 1,3:2,4-bis(3',4'-dimethylbenzylidene)sorbitol, 1,3:2,4-di(paramethyldibenzylidene)sorbitol, and 1,3:2,4-di(benzylidene)sorbitol.
11. Further fillers and reinforcing agents, for example calcium carbonate, silicates, glass fibres, glass bulbs, stainless steel fibres, aramide fibers, asbestos, talc, kaolin, mica, barium sulphate, metal oxides and hydroxides, carbon black, graphite, wood flour and flours or fibers of other natural products, synthetic fibres.
12. Other additives, for example blend compatibilizing agents, plasticisers, lubricants, emulsifiers, pigments, rheology additives, catalysts, flow-control agents, optical brighteners, flame proofing agents, antistatic agents and blowing agents.
13. Additional benzofuranones and indolinones, for example those disclosed in U.S. Patent Specification Nos. 4,325,863*;* 4,338,244*;* 5,175,312*;* 5,216,052*; or* 5,252,643*;* DE-A-4316611*;* DE-A-4316622*;* DE-A-4316876*;* EP-A-0589839 or EP-A-0591102 or 3-[4-(2-acetoxyethoxy)phenyl]-5,7-di-tert-butylbenzofuran-2-one, 5,7-di-tert-butyl-3-[4-(2-stearoyloxyethoxy)phenyl]benzofuran-2-one, 3,3'-bis[5,7-di-tert-butyl-3-(4-[2-hydroxyethoxy]phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butylbenzofuran-2-one, 3-(3,5-dimethyl-4-pivaloyloxyphenyl)-5,7-di-tert-butylbenzofuran-2-one, 3-(3,4-dimethylphenyl)-5,7-di-tert-butylbenzofuran-2-one, 3-(2,3-dimethylphenyl)-5,7-di-tert-butylbenzofuran-2-one.

Preferred additional additives for the compositions as defined above are processing stabilizers, such as the above-mentioned phosphites and phenolic antioxidants, and light stabilizers, such as benzotriazoles. Preferred specific antioxidants include octadecyl 3-(3,5-di-tert-butyl-4-hydroxyphenyl)-propionate (IRGANOX 1076). Specific processing stabilizers include tris(2,4-di-tert-butylphenyl)phosphite (IRGAFOS 168) and tetrakis(2,4-di-tert-butylphenyl)[1,1-biphenyl]-4,4'-diylbisphosphonite (IRGAFOS P-EPQ). Specific light stabilizers include 2-(2H-benzotriazole-2-yl)-4,6-bis(1-methyl-1-phenylethyl)phenol (TINUVIN 234), 2-(5-chloro(2H)-benzotriazole-2-yl)-4-(methyl)-6-(tert-butyl)phenol (TINUVIN 326), 2-(2H-benzotriazole-2-yl)-4-(1,1,3,3-tetramethylbutyl)phenol (TINUVIN 329), 2-(2H-benzotriazole-2-yl)-4-(tert-butyl)-6-(sec-butyl)phenol (TINUVIN 350), 2,2'-methylenebis(6-(2H-benzotriazol-2-yl)-4-(1,1,3,3-tetramethylbutyl)phenol) (TINUVIN 360), and 2-(4,6-diphenyl-1,3,5-triazin-2-yl)-5-[(hexyl)oxy]-phenol (TINUVIN 1577).

The additives mentioned above are preferably contained in an amount of 0.01 to 10.0%, especially 0.05 to 5.0%, relative to the weight of the polymer component b).

The incorporation of the additive component a) and optional further components into the polymer component b) is carried out by known methods such as dry blending in the form of a powder, or wet mixing in the form of solutions, dispersions or suspensions for example in an inert solvent, water or oil. The additive components a) and optional further additives may be incorporated, for example, before or after molding or also by applying the dissolved or dispersed additive or additive mixture to the polymer material, with or without subsequent evaporation of the solvent or the suspension/dispersion agent. They may be added directly into the processing apparatus (e.g. extruders, internal mixers, etc.), e.g. as a dry mixture or powder, or as a solution or dispersion or suspension or melt.

The addition of the additive components to the polymer substrate b) can be carried out in all customary mixing machines in which the polymer is melted and mixed with the additives. Suitable machines are known to those skilled in the art. They are predominantly mixers, kneaders and extruders.

The process is preferably carried out in an extruder by introducing the additive during processing.

Particularly preferred processing machines are single-screw extruders, contra rotating and co-rotating twin-screw extruders, planetary-gear extruders, ring extruders or co kneaders. It is also possible to use processing machines provided with at least one gas removal compartment to which a vacuum can be applied.

Suitable extruders and kneaders are described, for example, in Handbuch der Kunststoffextrusion, Vol. 1 Grundlagen, Editor F. Hensen, W. Knappe, H. Potente, 1989, pp. 3-7, ISBN:3-446-14339-4 *(*Vol. 2 Extrusionsanlagen 1986, ISBN 3-446-14329-7*).*

For example, the screw length is 1 - 60 screw diameters, preferably 35-48 screw diameters. The rotational speed of the screw is preferably 10 - 600 rotations per minute (rpm), preferably 25 - 300 rpm.

The maximum throughput is dependent on the screw diameter, the rotational speed and the driving force. The process of the present invention can also be carried out at a level lower than maximum throughput by varying the parameters mentioned or employing weighing machines delivering dosage amounts.

If a plurality of components is added, these can be premixed or added individually.

The additives component a) and optional further additives can also be sprayed onto the polymer substrate b). The additive mixture dilutes other additives, for example the conventional additives indicated above, or their melts so that they can be sprayed also together with these additives onto the polymer substrate. Addition by spraying during the deactivation of the polymerisation catalysts is particularly advantageous; in this case, the steam evolved may be used for deactivation of the catalyst. In the case of spherically polymerised polyolefins it may, for example, be advantageous to apply the additives of the invention, optionally together with other additives, by spraying.

The additive component a) and optional further additives can also be added to the polymer in the form of a master batch ("concentrate") which contains the components in a concentration of, for example, about 1.0% to about 40.0% and preferably 2.0% to about 20.0% by weight incorporated in a polymer. The polymer is not necessarily of identical structure than the polymer where the additives are added finally. In such operations, the polymer can be used in the form of powder, granules, solutions, and suspensions or in the form of lattices.

Incorporation can take place prior to or during the shaping operation. The materials containing the additives of the invention described herein preferably are used for the production of molded articles, for example roto-molded articles, injection molded articles, profiles and the like, and especially a fiber, spun melt non-woven, film or foam.

Thus, present invention further pertains to a molded or extruded article, a fiber, spun melt non-woven or a foam comprising the composition of the invention.

The following examples illustrate the invention:

### Examples

### Materials and Methods

PC 145 Resin (GE Plastics) is vacuum-dried for 8 h at 120 °C and stabilized with IRGAFOS® P-EPQ (Ciba Specialty Chemicals). Dyneon PA5931 (=PTFE) is used as an anti-dripping agent.

The polycarbonate compositions shown in Tab. 1 are extruded on a Haake TW-100 at 280°C and pelletized by strand granulation. After drying at 120°C for 12 h, the granulated compositions are injection molded at 290°C into plaques of 1.6 mm or 3.2 mm thickness according to Underwriter's Laboratories flame retardancy standard UL-94.

Flame retardancy is tested according to UL-94 in the vertical mode.

| Example | PC 145 Resin | IRGAFOS | FR | PTFE | UL-94 |
|---|---|---|---|---|---|
| 1 | 99.32 | 0.08 | 0.1 (1) | 0.5 | V-0 (1.6 mm) |
| 2 | 99.32 | 0.08 | 0.1 (2) | 0.5 | V-0 (1.6 mm) |
| 3 | 99.32 | 0.08 | 0.1 (3) | 0.5 | V-0 (1.6 mm) |
| 4* | 99.82 | 0.08 | 0.1 (1) | -- | V-0 (3.2 mm) |
| 5 | 99.57 | 0.08 | 0.1 (4) | 0.25 | V-0 (1.6 mm) |
| 6 | 99.32 | 0.08 | 0.1 (4) | 0.5 | V-0 (1.6 mm) |
| 7 | 99.57 | 0.08 | 0.1 (5) | 0.25 | V-0 (1.6 mm) |
| Comp. Ex. | 99.92 | 0.08 | -- | -- | V-2 (1.6 mm) |

| | | | | | |
|---|---|---|---|---|---|
| Flame Retardants (FR) present in composition: (1) TINOPAL SFP: Sodium-4,4'-bis(2-amino-4-monoethanolamino-6-s-triazinyl)-diaminostilbene-2,2'-disulphonate (structural formula above, cf. definitions of component a)) (2) TINOGARD HS: Sodium 3-(2H-benzotriazol-2-yl)-4-hydroxy-5-(1-methylpropyl)-benzene-sulphonate (structural formula above, cf. definitions of component a)) (3) TINOPAL CBS: Disodium 2,2'-(4,4'-biphenyldiyldi-2,1-ethenediyl)-dibenzenesulphonate (structural formula above, cf. definitions of component a)) (4) TINOGARD AO 6 (structural formula above, cf. definitions of component a)) (5) TINOGARD AO 6 K (potassium salt of TINOGARD AO 6, cf. definitions of component a) * Comparative Example | | | | | |

## Claims

1. A composition which comprises
a) At least one salt of an aromatic sulphonic acid selected from the group consisting of
a₁) Triazinylaminostilbene disulphonic acid salts of the formula 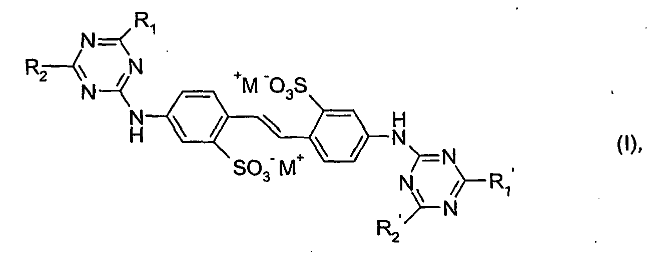 Wherein
R₁ and R₂ independently of one another represent -NH₂, -NHC₁-C₄alkyl,
-N(C₁-C₄alkyl)₂, -NH-hydroxy-C₂-C₄alkyl, -N(hydroxy-C₂-C₄alkyl)₂, -N(C₁-C₄alkyl)(hydroxy-C₂-C₄alkyl), phenylamino, diphenylamino, phenylamino or diphenylamino substituted by 1-3 substituents selected from the group consisting of -S(=O)₂-O⁻ M⁺, C₁-C₄alkyl and C₁-C₄alkoxy, or 5- or 6-membered saturated N-heterocyclyl;
R₁' and R₂' are as defined as R₁ and R₂; and
M⁺ represents an alkali metal atom, ammonium or a cation formed from an amine;
a₂) 2-(2-Hydroxyphenyl)-2H-benzotriazolylsulphonic acid salts of the formula 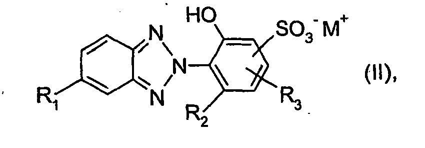 Wherein
R₁ represents hydrogen, C₁-C₄alkyl or the group -S(=O)₂-O⁻ M⁺;
R₂ represents hydrogen or hydroxy;
R₃ represents hydrogen, C₁-C₄alkyl, C₁-C₄alkoxy or benzyl; and
M⁺ represents an alkali metal atom, ammonium or a cation formed from an amine;
a₃) Sulphophenyl substituted bis-stilbene derivatives of the formula 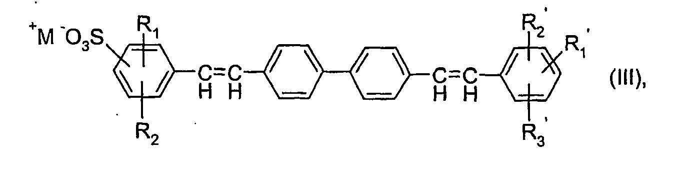 Wherein
R₁ and R₂ independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy;
R₁', R₂' and R₃' independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy; and
M⁺ represents an alkali metal atom, ammonium or a cation formed from an amine; and
a₄) Sulpho substituted 2,5-bis-(benzoxazolyl)-thiophene derivatives of the formula: 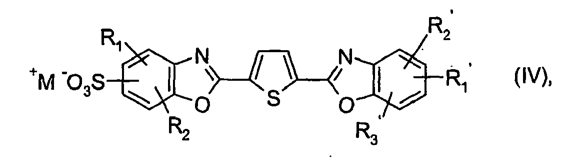 Wherein
R₁ and R₂ independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy;
R₁', R₂ and R₃' R₁ and R₂ independently of one another represent hydrogen or a substituent selected from the group consisting of -S(=O)₂-O⁻ M⁺, C₁-C₄alkyl, phenyl-C₁-C₄alkyl, hydroxy and C₁-C₄alkoxy; and
M⁺ represents an alkali metal atom, ammonium or a cation formed from an amine;
a₅) Sulpho substituted phenol derivatives of the formula 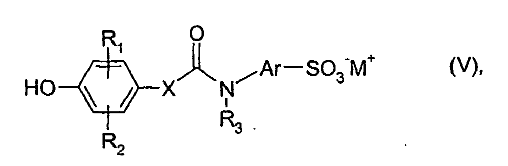 Wherein
R₁ and R₂ independently of one another represent hydrogen or a substituent selected from the group consisting of C₁-C₄alkyl, C₅-C₆cycloalkyl, C₅-C₆cycloalkyl-C₁-C₄alkyl, aryl and aryl-C₁-C₄alkyl;
R₃ represents hydrogen or C₁-C₄alkyl;
X represents C₂-C₆alkylene;
Ar represents phenyl, phenyl substituted by 1 or 2 substituents selected from the group consisting of C₁-C₄alkyl, C₅-C₆cycloalkyl, C₅-C₆cycloalkyl-C₁-C₄alkyl, aryl and aryl-C₁-C₄alkyl, naphthyl or naphthyl substituted by 1 - 4 C₁-C₄alkyl; and
M⁺ represents an alkali metal atom, ammonium or a cation formed from an amine; and as additional component anti-dripping agents; and
b) A polymer substrate comprising polycarbonate or polycarbonate blends.

2. A composition according to claim 1, which comprises polytetrafluoroethylene as an additional anti-dripping agent.

3. A composition according to claim 1 which comprises
a) At least one salt of an aromatic sulphonic acid selected from the group consisting of
a₁) Triazinylaminostilbene disulphonic acid salts (I), wherein
R₁ and R₂ independently of one another represent -NH₂, -N(C₁-C₄alkyl)₂, phenylamino substituted by 1-3 substituents selected from the group consisting of -S(=O)₂-O⁻ M⁺, C₁-C₄alkyl and C₁-C₄alkoxy, or 5- or 6-membered saturated N-heterocyclyl;
R₁' and R₂ are as defined as R₁ and R₂; and
M⁺ represents an alkali metal atom;
a₂) 2-(2-Hydroxyphenyl)-2H-benzotriazolylsulphonic acid salts (II), wherein
R₁ represents hydrogen;
R₂ represents hydrogen; and R₃ represents hydrogen or C₁-C₄alkyl; and
M⁺ represents an alkali metal atom;
a₃) Sulphophenyl substituted bis-stilbene derivatives (III), wherein
R₁ and R₂ independently of one another represent hydrogen or C₁-C₄alkyl; One of R₁', R₂' and R₃ represents -S(=O)₂-O⁻ M⁺ and the other ones represent hydrogen or C₁-C₄alkyl; and
M⁺ represents an alkali metal atom; and
a₄) Sulpho substituted 2,5-bis-(benzoxazolyl)-thiophene derivatives (IV), wherein
R₁ and R₂ independently of one another represent hydrogen or C₁-C₄alkyl; R₁', R₂ and R₃' independently of one another represent hydrogen or a substituent selected from the group consisting of -S(=O)₂-O⁻ M⁺ and C₁-C₄alkyl; and
M⁺ represents an alkali metal atom; and
a₅) Sulpho substituted phenol derivatives (V), wherein
R₁ and R₂ independently of one another represent hydrogen or C₁-C₄alkyl; R₃ represents hydrogen or C₁-C₄alkyl;
X represents C₂-C₆alkylene;
Ar represents phenyl or phenyl substituted by 1 or 2 C₁-C₄alkyl; and
M⁺ represents an alkali metal atom;
and
b) A polymer substrate comprising polycarbonate or polycarbonate blends.

4. A composition according to claim 1 which comprises
a) At least one salt of an aromatic sulphonic acid selected from the group consisting of
a₁) Triazinylaminostilbene disulphonic acid salts (I), wherein R₁ and R₂ independently of one another represent -N(C₁-C₄alkyl)₂, phenylamino substituted by 1 or 2 -S(=O)₂-O⁻ M⁺, or morpholinyl;
M⁺ represents an alkali metal atom; and
R₁' and R₂' are as defined as R₁ and R₂;
a₂) 2-(2-Hydroxyphenyl)-2H-benzotriazolylsulphonic acid salts (II), wherein
R₁ represents hydrogen or C₁-C₄alkyl;
R₂ represents hydrogen or hydroxy; and
R₃ represents hydrogen or C₁-C₄alkyl; and
M⁺ represents an alkali metal atom; and
a₃) Sulphophenyl substituted bis-stilbene derivatives (III), wherein
R₁ and R₂ independently of one another represent hydrogen or C₁-C₄alkyl;
One of R₁', R₂' and R₃' represents -S(=O)₂-O⁻ M⁺ and the other ones represent hydrogen or C₁-C₄alkyl; and
a₅) Sulpho substituted phenol derivatives (V), wherein
One of R₁ and R₂ represents methyl and the other one represents tert-butyl or both of R₁ and R₂ represent tert-butyl;
R₃ represents hydrogen or methyl;
X represents C₂-C₄alkylene;
Ar represents phenyl substituted by 1 methyl and 1 tert-butyl or by 2 tert-butyl; and
M⁺ represents an alkali metal atom; and
b) A polymer substrate comprising polycarbonate or polycarbonate blends.

5. A composition according to claim 1 which comprises
a) At least one salt of an aromatic sulphonic acid selected from the group consisting of
a₁) Triazinylaminostilbene disulphonic acid salts (I), wherein
One of R₁ and R₂ represents -N(C₁-C₄alkyl)₂ and the other one represents phenylamino substituted by 2 -S(=O)₂-O⁻ M⁺;
R₁' and R₂' are as defined as R₁ and R₂; and
M⁺ represents an alkali metal atom;
a₂) 2-(2-Hydroxyphenyl)-2H-benzotriazolylsulphonic acid salts (II), wherein
R₁ and R₂ represent hydrogen; and
R₃ represents hydrogen or C₁-C₄alkyl; and
M⁺ represents an alkali metal atom; and
a₃) Sulphophenyl substituted bis-stilbene derivatives (III), wherein
R₁ and R₂ represent hydrogen;
One of R₁', R₂' and R₃' represents -S(=O)₂-O⁻ M⁺ and the other ones represent hydrogen; and
M⁺ represents an alkali metal atom; and
a₅) Sulpho substituted phenol derivatives (V), wherein
R₁ and R₂ represent tert-butyl;
R₃ represents hydrogen;
X represents ethylene;
Ar represents phenyl substituted by 2 tert-butyl; and
M⁺ represents an alkali metal atom; and
b) A polymer substrate comprising polycarbonate or polycarbonate blends.

6. A composition according to claim 1 which comprises
a) At least one salt of an aromatic sulphonic acid selected from the group consisting of
a₁) A triazinylaminostilbene disulphonic acid salt of the formula 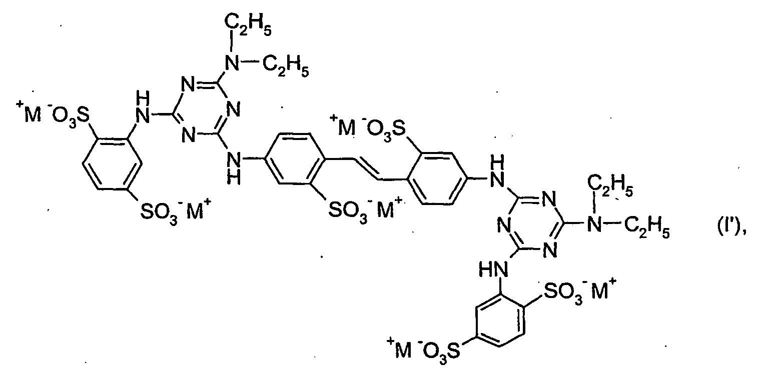
a₂) A 2-(2-hydroxyphenyl)-2H-benzotriazolylsulphonic acid salt of the formula 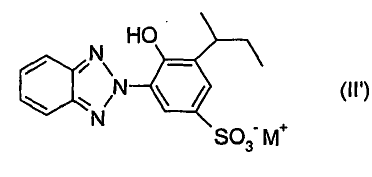 and
a₃) A sulphophenyl substituted bis-stilbene derivative of the formula 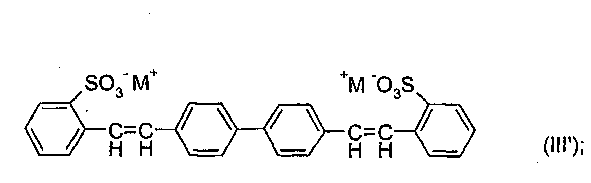 and
a₅) A salt of a sulpho substituted phenol derivative of the formula 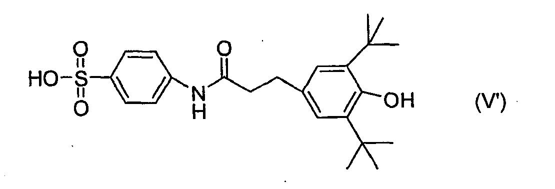 and
b) A polymer substrate comprising polycarbonate or polycarbonate blends.

7. A composition according to claim 1 which additionally comprises further additives selected from the group consisting of polymer stabilizers and additional flame-retardants.

8. A composition which comprises
a₅) Sulpho substituted phenol derivatives of the formula 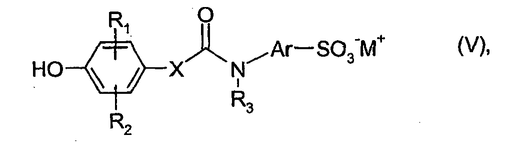 Wherein
R₁ and R₂ independently of one another represent hydrogen or a substituent selected from the group consisting of C₁-C₄alkyl, C₅-C₆cycloalkyl, C₅-C₆cycloalkyl-C₁-C₄alkyl, aryl and aryl-C₁-C₄alkyl;
R₃ represents hydrogen or C₁-C₄alkyl;
X represents C₂-C₆alkylene;
Ar represents phenyl, phenyl substituted by 1 or 2 substituents selected from the group consisting of C₁-C₄alkyl, C₅-C₆cycloalkyl, C₅-C₆cycloalkyl-C₁-C₄alkyl, aryl and aryl-C₁-C₄alkyl, naphthyl or naphthyl substituted by 1 - 4 C₁-C₄alkyl; and
M⁺ represents an alkali metal atom, ammonium or a cation formed from an amine; and;
b) A polymer substrate comprising polycarbonate or polycarbonate blends.

9. A process for imparting flame retardancy to a polymer substrate comprising polycarbonates or polycarbonate blends, which process comprises adding to said polymer substrate the salt of an aromatic sulphonic acid according to claim 1.

## Patentansprüche

1. Zusammensetzung, welche Folgendes umfasst:
a) mindestens ein Salz einer aromatischen Sulfonsäure, gewählt aus der Gruppe, die aus Folgendem besteht:
a₁) Triazinylaminostilbendisulfonsäuresalzen der Formel 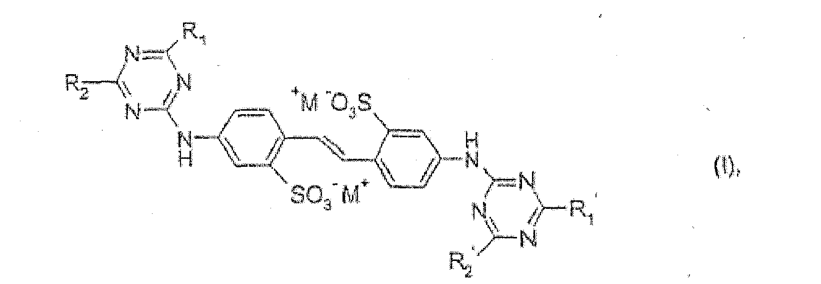 worin
R₁ und R₂ unabhängig voneinander für -NH₂, -NHC₁-C₄-Alkyl, -N(C₁-C₄-Alkyl)₂, -NH-Hydroxy-C₂-C₄-alkyl, -N(Hydroxy-C₂-C₄-alkyl)₂, -N(C₁-C₄-Alkyl) (hydroxy-C₂-C₄-alkyl), Phenylamino, Diphenylamino, Phenylamino oder Diphenylamino stehen, substituiert durch 1 - 3 Substituenten, die aus der Gruppe gewählt sind, die aus -S(=O)₂-O⁻M⁺, C₁-C₄-Alkyl und C₁-C₄-Alkoxy oder 5- oder 6-gliedrigem gesättigten N-Heterocyclyl besteht;
R₁' und R₂' so definiert sind wie R₁ und R₂; und
M⁺ für ein Alkalimetallatom, Ammonium oder ein Kation, gebildet aus einem Amin, steht;
a₂) 2-(2-Hydroxyphenyl)-2H-benzotriazolylsulfonsäuresalzen der Formel 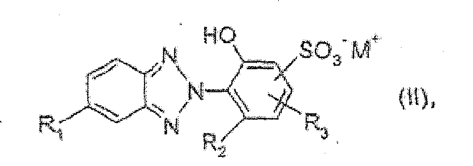 worin
R₁ für Wasserstoff, C₁-C₄-Alkyl oder die Gruppe -S(=O)₂-O⁻M⁺ steht;
R₂ für Wasserstoff oder Hydroxy steht;
R₃ für Wasserstoff, C₁-C₄-Alkyl oder C₁-C₄-Alkoxy oder -Benzyl steht; und
M⁺ für ein Alkalimetallatom, Ammonium oder ein Kation, gebildet aus einem Amin, steht;
a₃) Sulfophenyl-substituierten Bisstilbenderivaten der Formel 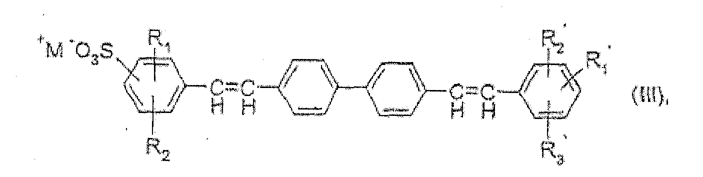 worin
R₁ und R₂ unabhängig voneinander für Wasserstoff oder einen Substituenten stehen, der aus der Gruppe gewählt ist, die aus -S(=O)₂-O⁻M⁺, C₁-C₄-Alkyl, Phenyl-C₁-C₄-alkyl, Hydroxy und C₁-C₄-Alkoxy besteht;
R₁', R₂' und R₃' unabhängig voneinander für Wasserstoff oder einen Substituenten stehen, der aus der Gruppe gewählt ist, die aus -S(=O)₂-O⁻M⁺, C₁-C₄-Alkyl, Phenyl-C₁-C₄-alkyl, Hydroxy und C₁-C₄-Alkoxy besteht; und
M⁺ für ein Alkalimetallatom, Ammonium oder ein Kation, gebildet aus einem Amin, steht; und
a₄) Sulfo-substituierten 2,5-Bis-(benzoxazolyl)-thiophenderivaten der Formel 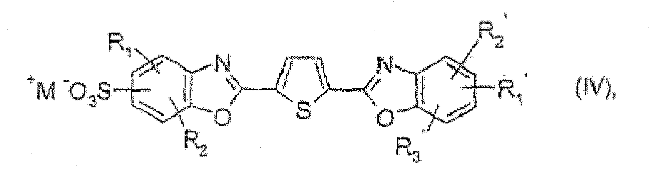 worin
R₁ und R₂ unabhängig voneinander für Wasserstoff oder einen Substituenten stehen, der aus der Gruppe gewählt ist, die aus -S(=O)₂-O⁻M⁺, C₁-C₄-Alkyl, Phenyl-C₁-C₄-alkyl, Hydroxy und C₁-C₄-Alkoxy besteht;
R₁', R₂' und R₃' unabhängig voneinander für Wasserstoff oder einen Substituenten stehen, der aus der Gruppe gewählt ist, die aus -S (=O)₂-O⁻M⁺, C₁-C₄-Alkyl, Phenyl-C₁-C₄-alkyl, Hydroxy und C₁-C₄-Alkoxy besteht; und
M⁺ für ein Alkalimetallatom, Ammonium oder ein Kation, gebildet aus einem Amin, steht;
a₅) Sulfo-substituierten Phenolderivaten der Formel 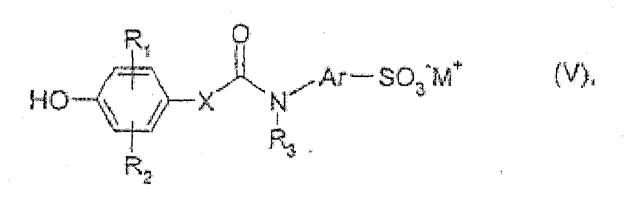 worin
R₁ und R₂ unabhängig voneinander für Wasserstoff oder einen Substituenten stehen, der aus der Gruppe gewählt ist, die aus C₁-C₄-Alkyl, C₅-C₆-Cycloalkyl, C₅-C₆-Cycloalkyl-C₁-C₄-alkyl, Aryl und Aryl-C₁-C₄-alkyl besteht;
R₃ für Wasserstoff oder C₁-C₄-Alkyl steht;
X für C₂-C₆-Alkylen steht;
Ar für Phenyl, Phenyl, substituiert durch 1 oder 2 Substituenten, die aus der Gruppe gewählt sind, die aus C₁-C₄-Alkyl, C₅-C₆-Cycloalkyl, C₅-C₆-Cycloalkyl-C₁-C₄-alkyl, Aryl und Aryl-C₁-C₄-alkyl besteht, Naphthyl oder Naphthyl, substituiert durch 1 - 4 C₁-C₄-Alkyl, steht; und
M⁺ für ein Alkalimetallatom, Ammonium oder ein Kation, gebildet aus einem Amin, steht; und als zusätzliche Komponente Antitropfmittel; und
b) ein Polymersubstrat, umfassend Polycarbonat oder Polycarbonatmischungen.

2. Zusammensetzung gemäß Anspruch 1, umfassend Polytetrafluorethylen als ein zusätzliches Antitropfmittel.

3. Zusammensetzung gemäß Anspruch 1, welche Folgendes umfasst:
a) mindestens ein Salz einer aromatischen Sulfonsäure, gewählt aus der Gruppe, die aus Folgendem besteht:
a₁) Triazinylaminostilbendisulfonsäuresalzen (I), wobei
R₁ und R₂ unabhängig voneinander für -NH₂, -N(C₁-C₄-Alkyl)₂, Phenylamino, substituiert durch 1 - 3 Substituenten, die aus der Gruppe gewählt sind, die aus -S(=O)₂-O⁻M⁺, C₁-C₄-Alkyl und C₁-C₄-Alkoxy besteht, oder 5- oder 6-gliedriges gesättigtes N-Heterocyclyl stehen;
R₁' und R₂' so definiert sind wie R₁ und R₂; und
M⁺ für ein Alkalimetallatom steht;
a₂) 2-(2-Hydroxyphenyl)-2H-benzotriazolylsulfonsäuresalzen (II), wobei
R₁ für Wasserstoff steht;
R₂ für Wasserstoff steht; und
R₃ für Wasserstoff oder C₁-C₄-Alkyl steht; und
M⁺ für ein Alkalimetallatom steht;
a₃) Sulfophenyl-substituierten
Bisstilbenderivaten (III), wobei
R₁ und R₂ unabhängig voneinander für Wasserstoff oder C₁-C₄-Alkyl stehen;
eines von R₁', R₂' und R₃' für -S(=O)₂-O⁻M⁺ steht und die anderen für Wasserstoff oder C₁-C₄-Alkyl stehen; und
M⁺ für ein Alkalimetallatom steht; und
a₄) Sulfo-substituierten 2,5-Bis-(benzoxazolyl)-thiophenderivaten (IV), wobei
R₁ und R₂ unabhängig voneinander für Wasserstoff oder C₁-C₄-Alkyl stehen;
R₁', R₂' und R₃' unabhängig voneinander für Wasserstoff oder einen Substituenten stehen, der aus der Gruppe gewählt ist, die aus -S(=O)₂-O⁻M⁺ und C₁-C₄-Alkyl besteht; und
M⁺ für ein Alkalimetallatom steht; und
a₅) Sulfo-substituierten Phenolderivaten (V), wobei
R₁ und R₂ unabhängig voneinander für Wasserstoff oder C₁-C₄-Alkyl stehen;
R₃ für Wasserstoff oder C₁-C₄-Alkyl steht;
X für C₂-C₆-Alkylen steht;
Ar für Phenyl oder Phenyl, substituiert durch 1 oder 2 C₁-C₄-Alkyl, steht;
M⁺ für ein Alkalimetallatom steht;
und
b) ein Polymersubstrat, umfassend Polycarbonat oder Polycarbonatmischungen.

4. Zusammensetzung gemäß Anspruch 1, welche Folgendes umfasst:
a) mindestens ein Salz einer aromatischen Sulfonsäure, gewählt aus der Gruppe, die aus Folgendem besteht:
a₁) Triazinylaminostilbendisulfonsäuresalzen (I), wobei
R₁ und R₂ unabhängig voneinander für -N(C₁-C₄-Alkyl)₂, Phenylamino, substituiert durch 1 oder 2 -S(=O)₂-O⁻M⁺, oder Morpholinyl stehen;
M⁺ für ein Alkalimetallatom steht; und
R₁' und R₂' so definiert sind wie R₁ und R₂;
a₂) 2-(2-Hydroxyphenyl)-2H-benzotriazolylsulfonsäuresalzen (II), wobei
R₁ für Wasserstoff oder C₁-C₄-Alkyl steht;
R₂ für Wasserstoff oder Hydroxy steht; und
R₃ für Wasserstoff oder C₁-C₄-Alkyl steht; und
M⁺ für ein Alkalimetallatom steht; und
a₃) Sulfophenyl-substituierten Bisstilbenderivaten (III), wobei
R₁ und R₂ unabhängig voneinander für Wasserstoff oder C₁-C₄-Alkyl stehen;
eines von R₁', R₂' und R₃' für -S (=O)₂-O⁻M⁺ steht und die anderen für Wasserstoff oder C₁-C₄-Alkyl stehen; und
a₅) Sulfo-substituierten Phenolderivaten (V), wobei
eines von R₁ und R₂ für Methyl steht und das andere für tert-Butyl steht oder beide aus R₁ und R₂ für tert-Butyl stehen;
R₃ für Wasserstoff oder Methyl steht;
X für C₂-C₄-Alkylen steht;
Ar für Phenyl, substituiert durch 1 Methyl und 1 tert-Butyl oder durch 2-tert-Butyl, steht; und
M⁺ für ein Alkalimetallatom steht; und
b) ein Polymersubstrat, umfassend Polycarbonat oder Polycarbonatmischungen.

5. Zusammensetzung gemäß Anspruch 1, die Folgendes umfasst:
a) mindestens ein Salz einer aromatischen Sulfonsäure, gewählt aus der Gruppe, die aus Folgendem besteht:
a₁) Triazinylaminostilbendisulfonsäuresalzen (I), wobei
eines aus R₁ und R₂ für -N(C₁-C₄-Alkyl)₂ steht und das andere für Phenylamino, substituiert durch 2 -S(=O)₂-O⁻M⁺, steht;
R₁' und R₂' so definiert sind wie R₁ und R₂; und
M⁺ für ein Alkalimetallatom steht;
a₂) 2-(2-Hydroxyphenyl)-2H-benzotriazolylsulfonsäuresalzen (II), wobei
R₁ und R₂ für Wasserstoff stehen; und
R₃ für Wasserstoff oder C₁-C₄-Alkyl steht; und
M⁺ für ein Alkalimetallatom steht; und
a₃) Sulfophenyl-substituierten Bisstilbenderivaten (III), wobei
R₁ und R₂ für Wasserstoff stehen;
eines von R₁', R₂' und R₃' für -S(=O)₂-O⁻M⁺ steht und die anderen für Wasserstoff stehen; und
M⁺ für ein Alkalimetallatom steht; und
a₅) Sulfo-substituierten Phenolderivaten (V), wobei
R₁ und R₂ für tert-Butyl stehen;
R₃ für Wasserstoff steht;
X für Ethylen steht;
Ar für Phenyl, substituiert durch 2-tert-Butyl, steht; und
M⁺ für ein Alkalimetallatom steht; und
b) ein Polymersubstrat, umfassend Polycarbonat oder Polycarbonatmischungen.

6. Zusammensetzung gemäß Anspruch 1, welche Folgendes umfasst:
a) mindestens ein Salz einer aromatischen Sulfonsäure, gewählt aus der Gruppe, die aus Folgendem besteht:
a₁) einem Triazinylaminostilbendisulfonsäuresalz der Formel: 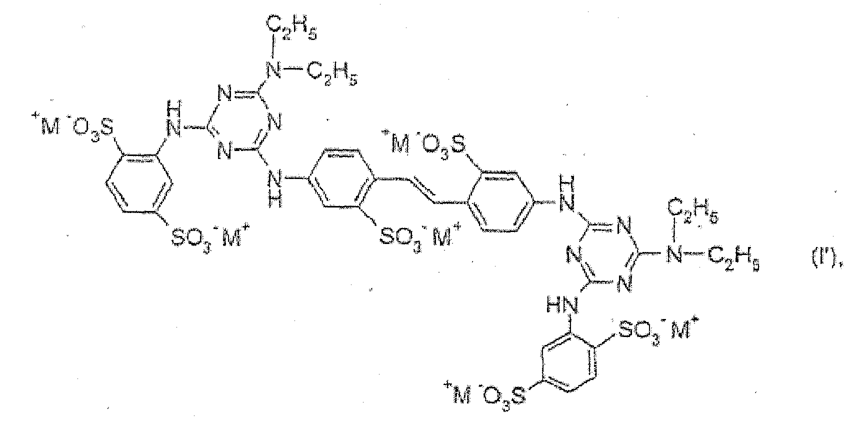
a₂) einem 2-(2-Hydroxyphenyl)-2H-benzotriazolylsulfonsäuresalz der Formel: 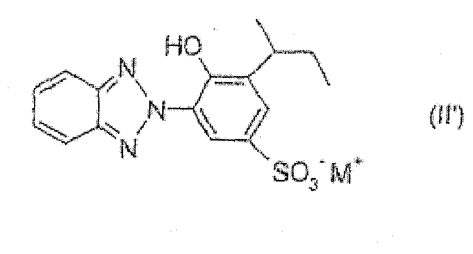 und
a₃) einem Sulfophenyl-substituierten Bisstilbenderivat der Formel: 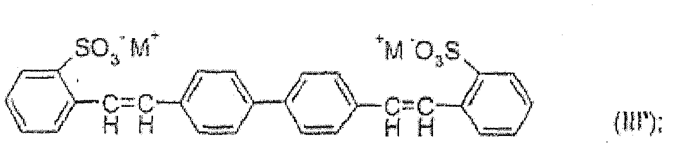 und
a₅) einem Salz eines Sulfo-substituierten Phenolderivats der Formel: 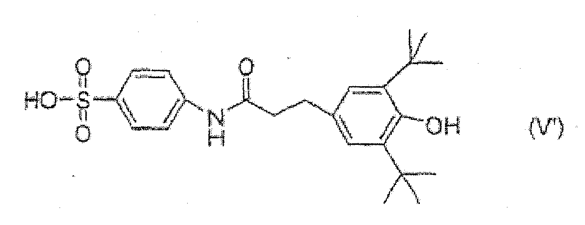 und
b) ein Polymersubstrat, umfassend Polycarbonat oder Polycarbonatmischungen.

7. Zusammensetzung gemäß Anspruch 1, welche zusätzlich weitere Additive umfasst, gewählt aus der Gruppe, die aus Polymerstabilisatoren und zusätzlichen flammenhemmenden Mitteln besteht.

8. Zusammensetzung, welche Folgendes umfasst:
a₅)Sulfo-substituierte Phenolderivate der Formel 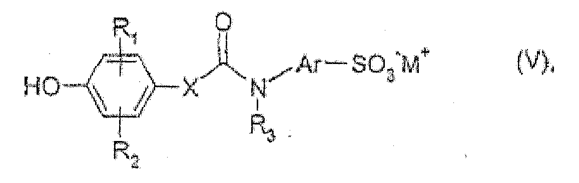 worin R₁ und R₂ unabhängig voneinander für Wasserstoff oder einen Substituenten stehen, der aus der Gruppe gewählt ist, die aus C₁-C₄-Alkyl, C₅-C₆-Cycloalkyl, C₅-C₆-Cycloalkyl-C₁-C₄-alkyl, Aryl und Aryl-C₁-C₄-alkyl besteht;
R₃ für Wasserstoff oder C₁-C₄-Alkyl steht;
X für C₂-C₆-Alkylen steht;
Ar für Phenyl, Phenyl, substituiert durch 1 oder 2 Substituenten, die aus der Gruppe gewählt sind, die aus C₁-C₄-Alkyl, C₅-C₆-Cycloalkyl, C₅-C₆-Cycloalkyl-C₁-C₄-alkyl, Aryl und Aryl-C₁-C₄-alkyl besteht, Naphthyl oder Naphthyl, substituiert durch 1 - 4 C₁-C₄-Alkyl, steht; und
M⁺ für ein Alkalimetallatom, Ammonium oder ein Kation, gebildet aus einem Amin, steht; und
b) ein Polymersubstrat, umfassend Polycarbonat oder Polycarbonatmischungen.

9. Verfahren zur Verleihung von Flammverzögerungsvermögen bei einem Polymersubstrat, welches Polycarbonate oder Polycarbonatmischungen umfasst, wobei das Verfahren das Hinzusetzen des Salzes einer aromatischen Sulfonsäure gemäß Anspruch 1 zu dem Polymersubstrat umfasst.

## Revendications

1. Composition qui comprend
a) au moins un sel d'un acide sulfonique aromatique choisi dans le groupe constitué par
a₁) les sels d'acide triazinylaminostilbènedisulfonique de formule 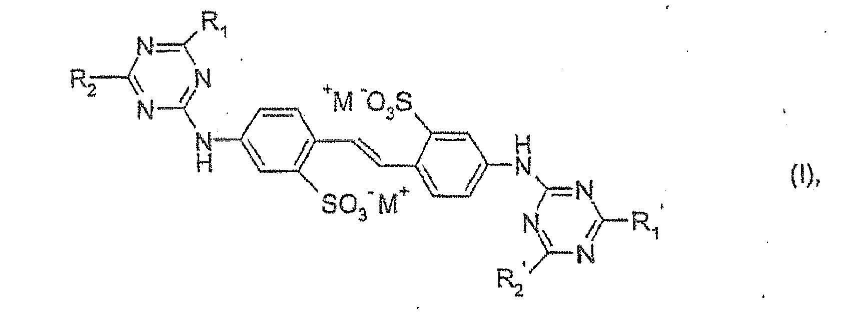 dans laquelle
R₁ et R₂ représentent, indépendamment l'un de l'autre, un groupe -NH₂, -NH-alkyle en C₁-C₄, -N(alkyle en C₁-C₄)₂, -NH-hydroxy-alkyle en C₂-C₄, -N(hydroxyalkyle en C₂-C₄)₂, -N(alkyle en C₁-C₄) (hydroxyalkyle en C₂-C₄), phénylamino, diphénylamino, phénylamino ou diphénylamino substitué par 1-3 substituants choisis dans le groupe constitué par -S(=O)₂-O⁻M⁺, alkyle en C₁-C₄ et alcoxy en C₁-C₄, ou N-hétérocyclyle saturé à 5 ou 6 chaînons ;
R₁' et R₂' sont tels que définis pour R₁ et R₂ ; et
M⁺ représente un atome de métal alcalin, un ammonium ou un cation formé à partir d'une amine ;
a₂) les sels d'acide 2-(2-hydroxyphényl)-2H-benzotriazolylsulfonique de formule 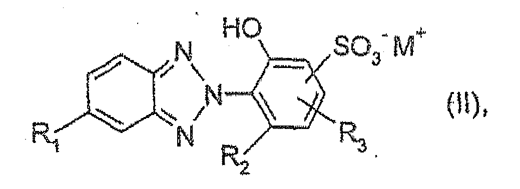 dans laquelle
R₁ représente un atome d'hydrogène, ou un groupe alkyle en C₁-C₄ ou le groupe -S(=O)₂-O⁻M⁺ ;
R₂ représente un atome d'hydrogène ou un groupe hydroxy ;
R₃ représente un atome d'hydrogène, ou un groupe alkyle en C₁-C₄, alcoxy en C₁-C₄ ou benzyle ; et
M⁺ représente un atome de métal alcalin, un ammonium ou un cation formé à partir d'une amine ;
a₃) les dérivés bis-stilbène à substitution sulfophényle de formule 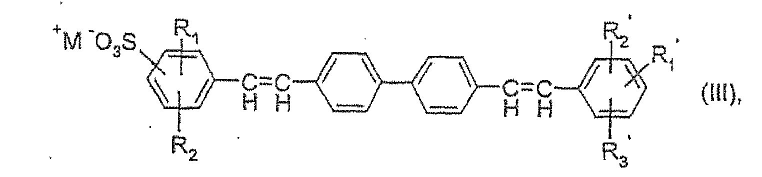 dans laquelle
R₁ et R₂ représentent, indépendamment l'un de l'autre, un atome d'hydrogène ou un substituant choisi dans le groupe constitué par -S(=O)₂-O⁻M⁺, alkyle en C₁-C₄, phényl-alkyle en C₁-C₄, hydroxy et alcoxy en C₁-C₄ ;
R₁', R₂' et R₃ ' représentent, indépendamment l'un de l'autre, un atome d'hydrogène ou un substituant choisi dans le groupe constitué par -S(=O)₂-O⁻M⁺, alkyle en C₁-C₄, phényl-alkyle en C₁-C₄, hydroxy et alcoxy en C₁-C₄ ; et
M⁺ représente un atome de métal alcalin, un ammonium ou un cation formé à partir d'une amine ; et
a₄) les dérivés 2,5-bis-(benzoxazolyl)thiophène à substitution sulfo de formule: 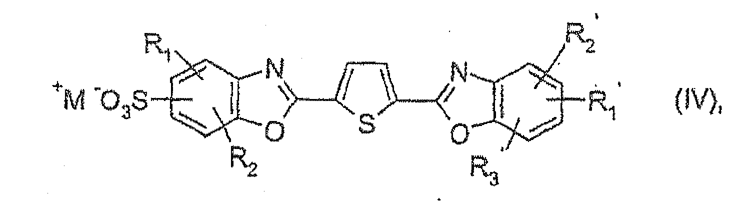 dans laquelle
R₁ et R₂ représentent, indépendamment l'un de l'autre, un atome d'hydrogène ou un substituant choisi dans le groupe constitué par -S(=O)₂-O⁻M⁺, alkyle en C₁-C₄, phényl-alkyle en C₁-C₄, hydroxy et alcoxy en C₁-C₄ ;
R₁', R₂' et R₃', R₁ et R₂ représentent, indépendamment l'un de l'autre, un atome d'hydrogène ou un substituant choisi dans le groupe constitué par -S(=O)₂-O⁻M⁺ alkyle en C₁-C₄, phényl-alkyle en C₁-C₄, hydroxy et alcoxy en C₁-C₄; et
M⁺ représente un atome de métal alcalin, un ammonium ou un cation formé à partir d'une amine ;
a₅) les dérivés phénol à substitution sulfo de formule 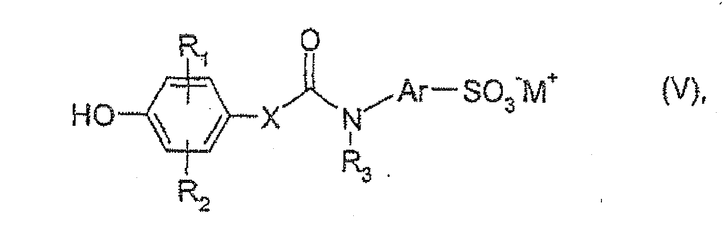 dans laquelle
R₁ et R₂ représentent, indépendamment l'un de l'autre, un atome d'hydrogène ou un substituant choisi dans le groupe constitué par les groupes alkyle en C₁-C₄, cycloalkyle en C₅-C₆, cycloalkyl(en C₅-C₆) -alkyle en C₁-C₄, aryle et aryl-alkyle en C₁-C₄ ;
R₂ représente un atome d'hydrogène ou un groupe alkyle en C₁-C₄;
X représente un groupe alkylène en C₂-C₆ ;
Ar représente un groupe phényle, phényle substitué par 1 ou 2 substituants choisis dans le groupe constitué par les groupes alkyle en C₁-C₄, cycloalkyle en C₅-C₆, cycloalkyl(en C₅-C₆) -alkyle en C₁-C₄, aryle et aryl-alkyle en C₁-C₄, naphtyle ou naphtyle substitué par 1 - 4 groupes alkyle en C₁-C₄ ; et
M⁺ représente un atome de métal alcalin, un ammonium ou un cation formé à partir d'une amine ; et comme constituant supplémentaire, des agents contre la chute des gouttes ; et
b) un substrat polymère comprenant un polycarbonate ou des mélanges de polycarbonates.

2. Composition selon la revendication 1, qui comprend du polytétrafluoroéthylène comme agent supplémentaire contre la chute des gouttes.

3. Composition selon la revendication 1 qui comprend
a) au moins un sel d'un acide sulfonique aromatique choisi dans le groupe constitué par
a₁) les sels d'acide triazinylaminostilbènedisulfonique (I), dans lesquels
R₁ et R₂ représentent, indépendamment l'un de l'autre, un groupe -NH₂, -N(alkyle en C₁-C₄)₂, phényl-amino substitué par 1-3 substituants choisis dans le groupe constitué par -S(=O)₂-O⁻M⁺, alkyle en C₁-C₄ et alcoxy en C₁-C₄, ou N-hétérocyclyle saturé à 5 ou 6 chaînons ; R₁' et R₂' sont tels que définis pour R₁ et R₂ ; et
M⁺ représente un atome de métal alcalin ;
a₂) les sels d'acide 2-(2-hydroxyphényl)-2H-benzotriazolylsulfonique (II), dans lesquels
R₁ représente un atome d'hydrogène ;
R₂ représente un atome d'hydrogène ; et
R₃ représente un atome d'hydrogène ou un groupe alkyle en C₁-C₄ ; et
M⁺ représente un atome de métal alcalin ;
a₃) les dérivés bis-stilbène à substitution sulfophényle (III), dans lesquels
R₁ et R₂ représentent, indépendamment l'un de l'autre, un atome d'hydrogène ou un groupe alkyle en C₁-C₄ ;
un des radicaux R₁', R₂' et R₃' représente -S(=O)₂-O⁻M⁺ et les autres représentent un atome d'hydrogène ou un groupe alkyle en C₁-C₄ ; et
M⁺ représente un atome de métal alcalin ; et
a₄) les dérivés 2,5-bis-(benzoxazolyl)thiophène à substitution sulfo (IV), dans lesquels
R₁ et R₂ représentent, indépendamment l'un de l'autre, un atome d'hydrogène ou un groupe alkyle en C₁-C₄ ;
R₁', R₂' et R₃' représentent, indépendamment l'un de l'autre, un atome d'hydrogène ou un substituant choisi dans le groupe constitué par -S(=O)₂-O⁻M⁺ et alkyle en C₁-C₄ ; et
M⁺ représente un atome de métal alcalin ; et
a₅) les dérivés phénol à substitution sulfo (V), dans lesquels
R₁ et R₂ représentent, indépendamment l'un de l'autre, un atome d'hydrogène ou un groupe alkyle en C₁-C₄ ;
R₃ représente un atome d'hydrogène ou un groupe alkyle en C₁-C₄ ;
X représente un groupe alkylène en C₂-C₆ ;
Ar représente un groupe phényle ou phényle substitué par 1 ou 2 groupes alkyle en C₁-C₄ ; et
M⁺ représente un atome de métal alcalin ;
et
b) un substrat polymère comprenant un polycarbonate ou des mélanges de polycarbonates.

4. Composition selon la revendication 1 qui comprend
a) au moins un sel d'un acide sulfonique aromatique choisi dans le groupe constitué par
a₁) les sels d'acide triazinylaminostilbènedisulfonique (I), dans lesquels
R₁ et R₂ représentent, indépendamment l'un de l'autre, un groupe -N(alkyle en C₁-C₄)₂, phénylamino substitué par 1 ou 2 groupes -S(=O)₂-O⁻M⁺, ou morpholinyle ;
M⁺ représente un atome de métal alcalin ; et R₁' et R₂' sont tels que définis pour R₁ et R₂ ;
a₂) les sels d'acide 2-(2-hydroxyphényl)-2H-benzotriazolylsulfonique (II), dans lesquels
R₁ représente un atome d'hydrogène ou un groupe alkyle en C₁-C₄ ;
R₂ représente un atome d'hydrogène ou un groupe hydroxy ; et
R₃ représente un atome d'hydrogène ou un groupe alkyle en C₁-C₄ ; et
M⁺ représente un atome de métal alcalin ; et a₃) les dérivés bis-stilbène à substitution sulfophényle (III), dans lesquels
R₁ et R₂ représentent, indépendamment l'un de l'autre, un atome d'hydrogène ou un groupe alkyle en C₁-C₄ ;
un des radicaux R₁', R₂' et R₃' représente -S(=O)₂-O⁻M⁺ et les autres représentent un atome d'hydrogène ou un groupe alkyle en C₁-C₄ ; et
a₅) les dérivés phénol à substitution sulfo (V), dans lesquels
un des radicaux R₁ et R₂ représente un groupe méthyle et l'autre représente un groupe t-butyle ou les deux radicaux R₁ et R₂ représentent un groupe t-butyle ;
R₃ représente un atome d'hydrogène ou un groupe méthyle ;
X représente un groupe alkylène en C₂-C₄ ;
Ar représente un groupe phényle substitué par 1 groupe méthyle et 1 groupe t-butyle ou par 2 groupes t-butyle ; et
M⁺ représente un atome de métal alcalin ; et
b) un substrat polymère comprenant un polycarbonate ou des mélanges de polycarbonates.

5. Composition selon la revendication 1 qui comprend
a) au moins un sel d'un acide sulfonique aromatique choisi dans le groupe constitué par
a₁) les sels d'acide triazinylaminostilbènedisulfonique (I), dans lesquels
un des radicaux R₁ et R₂ représente un groupe -N(alkyle en C₁-C₄)₂ et l'autre représente un groupe phénylamino substitué par 2 groupes -S(-O)₂O⁻M⁺ ;
R₁' et R₂' sont tels que définis pour R₁ et R₂ ; et
M⁺ représente un atome de métal alcalin ;
a₂) les sels d'acide 2-(2-hydroxyphényl)-2H-benzotriazolylsulfonique (II), dans lesquels
R₁ et R₂ représentent un atome d'hydrogène ; et
R₃ représente un atome d'hydrogène ou un groupe alkyle en C₁-C₄ ; et
M⁺ représente un atome de métal alcalin ; et
a₃) les dérivés bis-stilbène à substitution sulfophényle (III), dans lesquels
R₁ et R₂ représentent un atome d'hydrogène ;
un des radicaux R₁', R₂' et R₃' représente -S(=O)₂-O⁻M⁺ et les autres représentent un atome d'hydrogène ; et
M⁺ représente un atome de métal alcalin ; et
a₅) les dérivés phénol à substitution sulfo (V), dans lesquels
R₁ et R₂ représentent un groupe t-butyle ;
R₃ représente un atome d'hydrogène ;
X représente un groupe éthylène ;
Ar représente un groupe phényle substitué par 2 groupes t-butyle ; et
M⁺ représente un atome de métal alcalin ; et
b) un substrat polymère comprenant un polycarbonate ou des mélanges de polycarbonates.

6. Composition selon la revendication 1 qui comprend
a) au moins un sel d'un acide sulfonique aromatique choisi dans le groupe constitué par
a₁) un sel d'acide triazinylaminostilbènedisulfonique de formule 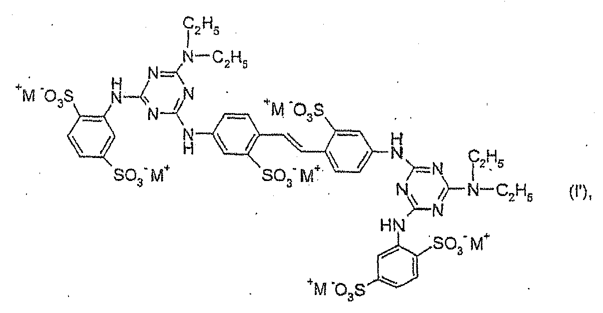
a₂) un sel d'acide 2-(2-hydroxyphényl)-2H-benzotriazolylsulfonique de formule 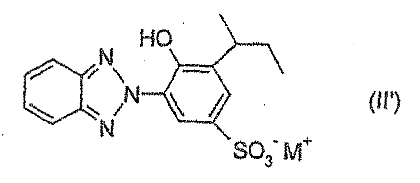 et
a₃) un dérivé bis-stilbène à substitution sulfophényle de formule 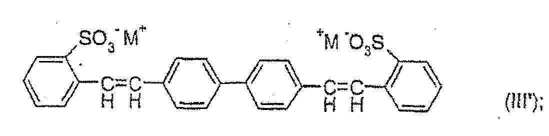 et
a₅) un sel d'un dérivé phénol à substitution sulfo de formule 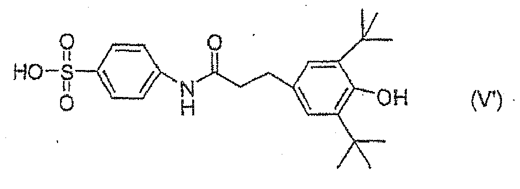 et
b) un substrat polymère comprenant un polycarbonate ou des mélanges de polycarbonates.

7. Composition selon la revendication 1 qui comprend en plus d'autres additifs choisis dans le groupe constitué par les stabilisants pour polymères et d'autres ignifugeants.

8. Composition qui comprend
a₅) des dérivés phénol à substitution sulfo de formule 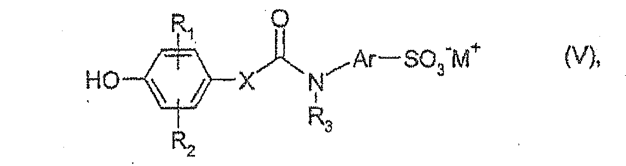 dans laquelle
R₁ et R₂ représentent, indépendamment l'un de l'autre, un atome d'hydrogène ou un substituant choisi dans le groupe constitué par les groupes alkyle en C₁-C₄, cycloalkyle en C₅-C₆, cycloalkyl (en C₅-C₆)-alkyle en C₁-C₄, aryle et aryl-alkyle en C₁-C₄ ;
R₃ représente un atome d'hydrogène ou un groupe alkyle en C₁-C₄ ;
X représente un groupe alkylène en C₂-C₆ ;
Ar représente un groupe phényle, phényle substitué par 1 ou 2 substituants choisis dans le groupe constitué par les groupes alkyle en C₁-C₄, cycloalkyle en C₅-C₆, cycloalkyl(en C₅-C₆) -alkyle en C₁-C₄, aryle et aryl-alkyle en C₁-C₄, naphtyle ou naphtyle substitué par 1 - 4 groupes alkyle en C₁-C₄; et
M⁺ représente un atome de métal alcalin, un ammonium ou un cation formé à partir d'une amine ; et
b) un substrat polymère comprenant un polycarbonate ou des mélanges de polycarbonates.

9. Procédé d'ignifugation d'un substrat polymère comprenant des polycarbonates ou des mélanges de polycarbonates, ce procédé comprenant l'addition audit substrat polymère du sel d'un acide sulfonique aromatique selon la revendication 1.
